# EUROPÄISCHE PATENTANMELDUNG

(11) **EP 4 298 908 A2**
(43) Veröffentlichungstag der Anmeldung: **03.01.2024**
(21) Anmeldenummer: 23204176.4
(22) Anmeldetag: 26.03.2018
(51) Int. Cl.: A01N 43/78

(54) **TRIZYKLISCHE CARBOXAMIDE ZUR BEKÄMPFUNG VON ARTHROPODEN**

(30) Priorität: 31.03.2017 EP 17164442
(62) Teilanmeldung aus: 18711973.0
(71) Anmelder: Bayer Animal Health GmbH, 51373 Leverkusen (DE)
(72) Erfinder: HARSCHNECK, Tobias, 40223 Düsseldorf (DE); ARLT, Alexander, 50859 Köln (DE); VELTEN, Robert, 40764 Langenfeld (DE); MAUE, Michael, 40764 Langenfeld (DE); ILG, Kerstin, 50670 Köln (DE); HALLENBACH, Werner, 40789 Monheim (DE); GÖRGENS, Ulrich, 40882 Ratingen (DE); HORSTMANN, Sebastian, 51381 Leverkusen (DE); LÖSEL, Peter, 51371 Leverkusen (DE); TURBERG, Andreas, 42781 Haan (DE)
(74) Vertreter: Cohausz & Florack

(57) **Zusammenfassung**

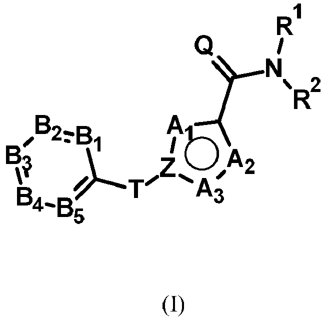

Die Erfindung umfasst unter anderem Verbindungen der allgemeinen Formel (I). Ferner werden Verfahren zur Herstellung der Verbindungen der Formel (I) beschrieben. Die erfindungsgemäßen Verbindungen eignen sich insbesondere zur Bekämpfung von Insekten, Spinnentieren und Nematoden in der Landwirtschaft sowie von Ektoparasiten in der Veterinärmedizin.

## Beschreibung

### Einleitung

Die vorliegende Anmeldung betrifft neue Verbindungen, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt, dass bestimmte Halogen-substituierte Verbindungen insektizid wirksam sind (EP 1 911 751, WO2012/069366, WO2012/080376, WO2012/107434 und WO2012/175474).

WO 2011/113756 offenbart Triazol Derivate, die insektizidale Wirkung aufweisen.

Ferner ist bekannt, dass bestimmte Halogen-substituierte Verbindungen Cytokin-inhibitorische Aktivitäten aufweisen (WO 2000/07980).

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass bestimmte Halogen-substituierte Verbindungen sowie deren Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Ähnliche Verbindungen sind bereits aus WO 2010/051926, WO2012/102387, WO2012/000896, WO2011/045224, WO2008/074835 bekannt geworden.

### Ausführungsformen der erfindungsgemäßen Verbindungen

Ein Aspekt der vorliegenden Erfindung bezieht sich auf neue insektizid, akarizid und/oder parasitizid wirksame Halogen-substituierte Verbindungen der allgemeine Formel (I) 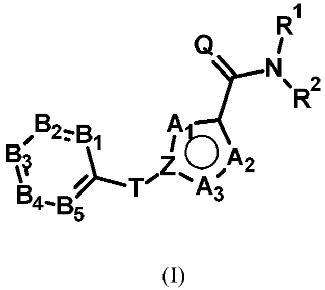 worin
- R¹: für H, jeweils gegebenenfalls mit einem oder mehreren Substitutenten unabhängig voneinander ausgewählt aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N*,*N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N*,*N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N*,*N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden (z. B. Methyliden oder Ethyliden), einer Oxogruppe oder einer Iminogruppe substituiertes C₁-C₆-Alkyl, C₂-C₆-Alkenyl, C₂-C₆-Alkinyl, C₃-C₇-Cycloalkyy, C₁-C₆-Alkylcarbonyl, C₁-C₆-Alkoxycarbonyl, C₆-,C₁₀-,C₁₄-Aryl(C₁-C₃)-alkyl, 3, 4, 5, 6, 7, 8, 9 oder 10-gliedrigen-Heterocyclyl (z. B. Thietanyl wie Thiethanyl-3-yl, Oxidothiethan wie 1-Oxido-thietan-3-yl, oder Dioxidothiethan wie 1,1-Dioxido-thietan-3-yl) oder 3, 4, 5, 6, 7, 8, 9 oder 10-gliedrigen-Heterocyclyl(C₁-C₃)-alkyl steht,
bevorzugt für gegebenenfalls mit Halogen oder Cyano substituiertem C₃-C₇-Cycloalkyl, oder
C₆-,C₁₀-,C₁₄-Aryl(C₁-C₃)-alkyl wobei C₆-,C₁₀-,C₁₄-Aryl gegebenenfalls mit einem oder mehreren Substitutenten unabhängig voneinander ausgewählt aus Amino, Hydroxy, Halogen, Nitro, Cyano, Isocyano, Mercapto und C₁-C₄-Alkyl substituiert sein kann, oder
C₁-C₆-Alkylcarbonyl;
mehr bevorzugt für gegebenenfalls mit Cyano substituiertem C₃-Cycloalkyl (insbesondere Cyclopropyl oder 1-CN-Cyclopropyl) oder C₆-Aryl(C₁-C₃)-alkyl (insbesondere -CH₂-C₆H₅);
- R²: für H oder gegebenenfalls mit einem oder mehreren Substitutenten unabhängig voneinander ausgewählt aus Amino, Hydroxy, Halogen, Nitro, Cyano, Mercapto, C₁-C₄-Carboxy, Carbonamid, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N*,*N*-Di-C₁-C₄-alkylamino, *N-*C₁*-*C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N*,*N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N*,*N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden (z. B. Methyliden oder Ethyliden) oder einer Oxogruppe substituiertes C₁-C₆-Alkyl steht (bevorzugt für H oder -CH₃);
die Gruppierungen
A₁, A₂ und A₃ unabhängig voneinander für N, O, CR³, S oder N-R⁴, bevorzugt für N, CR³, S oder N-R⁴ stehen, wobei A₁, A₂, A₃, Z und das C-Atom des Ringes ein aromatisches System bilden;
R³ jeweils unabhängig voneinander für H, Cl, F, I, Br oder gegebenenfalls halogeniertes C₁-C₄-Alkyl, bevorzugt Methyl steht;
R⁴ jeweils unabhängig für H oder gegebenenfalls halogeniertes C₁-C₄-Alkyl, bevorzugt Methyl steht;
Q für O oder S, bevorzugt O steht;
Z für C oder N steht; bevorzugt für C steht;
B₁ und B₅ unabhängig voneinander für N oder C-R⁵ und
B₂, B₃ und B₄ unabhängig voneinander für C-R⁵ stehen, wobei
R⁵ unabhängig voneinander für H, Halogen, Cyano, Nitro, SF₅, jeweils gegebenenfalls mit einem oder mehreren Substitutenten ausgewählt aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N-*Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-, C₁₀-, C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N-*Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N*,*N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden (z. B. Methyliden oder Ethyliden), einer Oxogruppe oder einer Iminogruppe substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N*,*N*-Di-C₁-C₆-alkylamino, steht;
T für einen der nachfolgend aufgeführten 5-gliedrigen Heteroaromaten T1-T4 und T6 steht, wobei die

Bindung zu 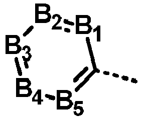 mit einer Raute # gekennzeichnet ist, 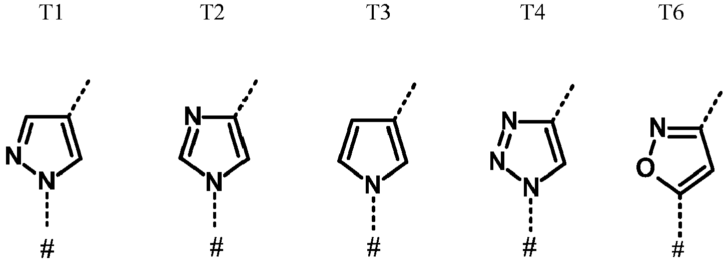 sowie Salze, N-Oxide und tautomere Formen der Verbindungen der Formel (I).

Eine bevorzugte Ausführungsform der vorliegenden Erfindung richtet sich dabei auf Verbindungen gemäß Absatz [0009], wobei R² für H oder Methyl steht.

Eine weitere bevorzugte Ausführungsform richtet sich dabei auf Verbindungen gemäß einem der Absätze [0009] oder [0010], wobei T für T1, T2 oder T4 steht, besonders bevorzugt für T1 steht.

Eine weitere bevorzugte Ausführungsform richtet sich dabei auf Verbindungen gemäß einem der Absätze [0009] bis [0011], wobei R¹ für gegebenenfalls mit Cyano substituiertes Cyclopropyl (bevorzugt Cyclopropyl oder 1-CN-Cyclopropyl) oder C₆-Aryl(C₁-C₃)-alkyl (bevorzugt-CH₂-C₆H₅) steht. Besonders bevorzugt steht R¹ für Benzyl, Cyclopropyl oder 1-CN-Cyclopropyl, ganz besonders bevorzugt für Cyclopropyl oder 1-CN-Cyclopropyl.

Eine weitere bevorzugte Ausführungsform richtet sich dabei auf Verbindungen gemäß einem der Absätze [0009] bis [0012], wobei Q für O steht.

Eine weitere bevorzugte Ausführungsform richtet sich dabei auf Verbindungen gemäß einem der Absätze [0009] bis [0013], wobei A₃ für S steht.

Eine weitere bevorzugte Ausführungsform richtet sich dabei auf Verbindungen gemäß einem der Absätze [0009] bis [0014], wobei A₃ für S steht, Z für C steht, A₂ für C-(gegebenenfalls halogeniertes C₁-C₄-alkyl) (bevorzugt C-CH₃), C-H oder C-Hal steht, worin Hal für Cl, Br, oder I, bevorzugt Br oder Cl, steht, und A₁ für C-H oder N steht. In einer weiteren bevorzugten Ausführungsform steht A₃ für S, Z für C, A₂ für C-Hal, worin Hal für Cl, Br, oder I steht, und A₁ für C-H steht. In einer weiteren bevorzugten Ausführungsform steht A₃ für S, Z für C, A₂ für C-H oder C-Hal, worin Hal für Cl, Br, oder I, bevorzugt Cl, steht, und A₁ für N steht.

Eine weitere bevorzugte Ausführungsform richtet sich dabei auf Verbindungen gemäß einem der Absätze [0009] bis [0013], wobei A₁ für S steht, bevorzugt worin A₁ für S steht, A₂ für C-H oder C-Hal, bevorzugt C-Hal, worin Hal für Cl, Br, oder I, bevorzugt Br oder Cl, noch mehr bevorzugt für Cl, steht Z für C steht, und A₃ für C-H steht.

Eine weitere bevorzugte Ausführungsform richtet sich dabei auf Verbindungen gemäß einem der Absätze [0009] bis [0013], wobei A₃ für O steht.

Eine weitere bevorzugte Ausführungsform richtet sich dabei auf Verbindungen gemäß einem der Absätze [0009] bis [0017], wobei A₃ für O steht, Z für C steht, A₁ für C-H steht und A₂ für C-Hal steht, worin Hal für Cl, Br, oder I, bevorzugt Cl, steht.

Eine weitere bevorzugte Ausführungsform richtet sich dabei auf Verbindungen gemäß einem der Absätze [0009] bis [0013], wobei A₃ für C-H steht, A₁ für S steht, Z für C steht und A₂ für C-Hal, worin Hal für Cl, Br, oder I, bevorzugt Cl, steht, steht.

Eine weitere bevorzugte Ausführungsform richtet sich dabei auf Verbindungen gemäß einem der Absätze [0009] bis [0013], wobei A₃ für C-H oder C-Hal steht, worin Hal für Cl, Br, oder I, bevorzugt Cl, steht, A₂ für N-H oder N-(C₁-C₄-alkyl) (bevorzugt für N-(C₁-C₄-alkyl), mehr bevorzugt für N-CH₃) steht, A₁ für C-H, C-Hal, worin Hal für Cl, Br, oder I, bevorzugt Cl, oder C-(C₁-C₄-Alkyl) steht, mehr bevorzugt worin A₃ für C-H oder C-Cl steht A₂ für N-CH₃ steht, A₁ für C-H steht.

Eine weitere bevorzugte Ausführungsform richtet sich dabei auf Verbindungen gemäß einem der Absätze [0009] bis [0013], wobei Z für C steht, A₁ für N steht, A₂ für S steht und A₃ für C-H steht.

Eine weitere bevorzugte Ausführungsform richtet sich dabei auf Verbindungen gemäß einem der Absätze [0009] bis zum vorherigen Absatz, worin B₁ und B₅ jeweils für C-H, C-CH₃ oder C-Hal, bevorzugt C-Cl, stehen. Besonders bevozugt worin B₁ und B₅ jeweils für C-Cl stehen.

Eine weitere bevorzugte Ausführungsform richtet sich dabei auf Verbindungen gemäß einem der Absätze [0009] bis zum vorherigen Absatz, worin B₂ und B₄ jeweils für C-H stehen.

Eine weitere bevorzugte Ausführungsform richtet sich dabei auf Verbindungen gemäß einem der Absätze [0009] bis zum vorherigen Absatz, worin B₃ für CR⁵ und R⁵ für perhalogeniertes C₁-C₄-Alkyl steht, bevorzugt für perfluoriertes C₁-C₄-Alkyl, mehr bevorzugt für perfluoriertes Propyl steht.

Eine weitere bevorzugte Ausführungsform richtet sich dabei auf Verbindungen der Formel (Ia) 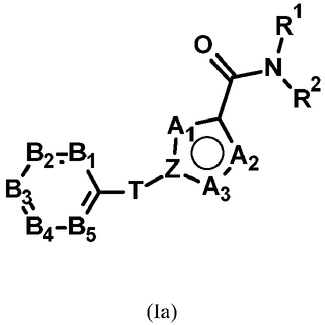 Worin B₁ bis B₅, A₁ bis A₃, Z, R¹, R² und T wie für Verbindungen der Formel (I) und ihre bevorzugten Ausführungsformen hierin definiert sind.

Eine weitere bevorzugte Ausführungsform richtet sich dabei auf Verbindungen der Formel (Ib) 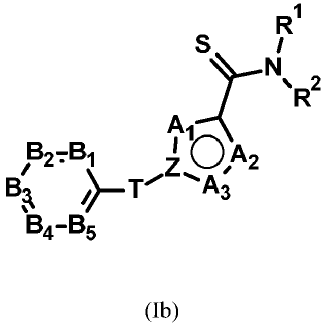 Worin Bi bis Bs, A₁ bis A₃, Z, R¹, R² und T wie für Verbindungen der Formel (I) und ihre bevorzugten Ausführungsformen hierin definiert sind.

Weitere bevorzugte Ausführungsformen richten sich jeweils auf Verbindungen der Formeln (1c), (1d) und (1f) bis (1k), in denen die jeweils gegebenenfalls vorkommenden Platzhalter A₁ bis A₃, B₁ bis B₅, T, Z, R¹ bis R⁴, wenn nicht für eine dieser Formeln explizit definiert, wie für Verbindungen der Formel (I) und ihre bevorzugten Ausführungsformen hierin definiert sind und "Alk" für C₁-C₄-Alkyl steht, "Hal" für Chlor oder Brom steht und V für C₁-C₄-Alkoxy oder N(R¹R²) steht.

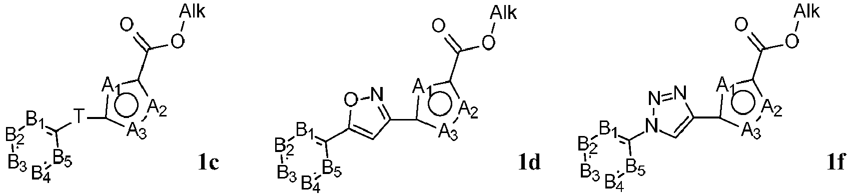

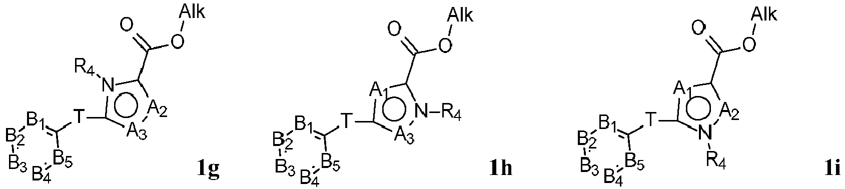

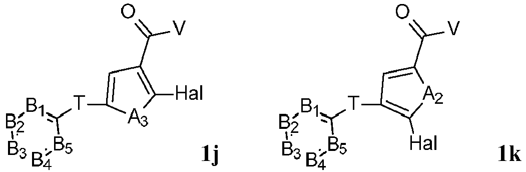

Eine weitere bevorzugte Ausführungsform richtet sich dabei auf Verbindungen der Formel (I`a) 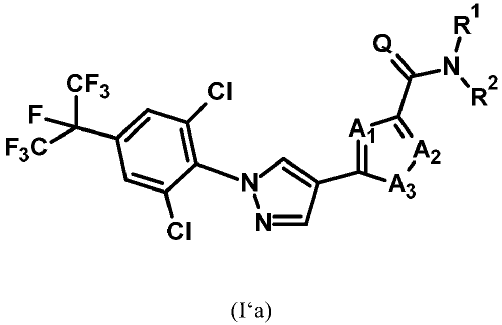 Worin Q, R¹, R², A₁, A₂ und A₃ wie für Verbindungen der Formel (I) und ihre bevorzugten Ausführungsformen hierin definiert sind.

Eine weitere bevorzugte Ausführungsform richtet sich dabei auf Verbindungen gemäß der Formel (I'b) 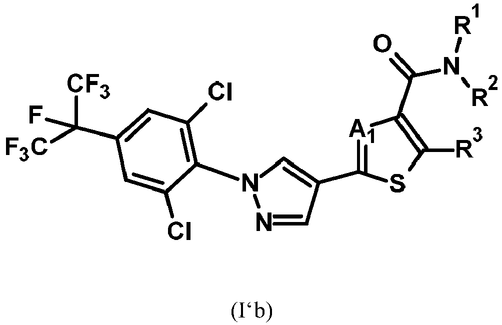 worin A₁ für N oder C-H steht und R³ wie hierin beschrieben definiert ist, bevorzugt für C₁-C₄-alkyl C-H oder C-Hal, worin Hal für Cl, Br, oder I, bevorzugt Br oder Cl, steht, steht, besonders bevorzugt R³ für CH₃ steht, und ansonsten R¹ und R² wie hierin beschrieben definiert sind.

Eine weitere bevorzugte Ausführungsform richtet sich dabei auf Verbindungen gemäß der Formel (I` c) 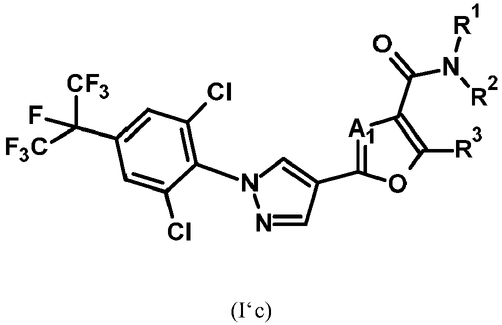 worin A₁ für C-H steht und R₃ bevorzugt für C-Cl steht und ansonsten R¹ und R² wie hierin beschrieben definiert sind.

Eine weitere bevorzugte Ausführungsform richtet sich dabei auf Verbindungen gemäß der Formel (I'd) 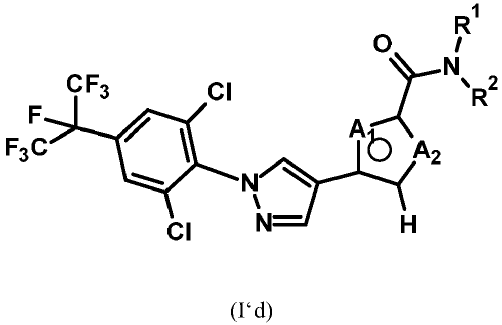 Worin A₁ für C-R³, bevorzugt für C-H, steht wenn A₂ für N-(halogeniertes C₁-C₄-Alkyl) oder N-(C₁-C₄-Alkyl) steht; oder A₁ für N steht wenn A₂ für S steht und ansonsten R¹ und R² wie hierin beschrieben definiert sind.

Bevorzugt steht R² in einer der Formeln (I'a), (I'b), (I'c) und (I'd) für H oder CH₃ und R¹ für gegebenenfalls mit CN substituiertes Cyclopropyl (wie Cyclopropyl oder 1-CN-Cyclopropyl).

Ein weiterer Aspekt bezieht sich auf ein insektizides Mittel umfassend mindestens eine Verbindung der Formel (I) oder einer von Formel (I) abgeleiteten Formel gemäß einem der Absätze [0009] bis zum vorherigen Absatz und einem Streckmittel und/oder einer oberflächenaktiven Substanz.

Ein weiterer Aspekt bezieht sich auf ein Verfahren zum Schutz von transgenem oder konventionellem Saatgut und der daraus entstehenden Pflanze vor dem Befall von Schädlingen, dadurch gekennzeichnet, dass das Saatgut mit mindestens einer Verbindung gemäß einem der Absätze [0009] bis [0032] behandelt wird.

Ein weiterer Aspekt bezieht sich auf die Verwendung von Verbindungen gemäß einem der Absätze [0009] bis [0032], oder von einem insektiziden Mittel gemäß Absatz [0033] zum Bekämpfen von Schädlingen.

Ein weiterer Aspekt bezieht sich auf Saatgut, bei dem eine Verbindung gemäß einem der Absätze [0009] bis [0032] als Bestandteil einer Umhüllung oder als weitere Schicht oder weitere Schichten zusätzlich zu einer Umhüllung auf das Saatgut aufgebracht ist.

Ein weiterer Aspekt betrifft ein Verfahren zum Schutz von transgenem oder konventionellem Saatgut und der daraus entstehenden Pflanze vor dem Befall von Schädlingen, dadurch gekennzeichnet, dass das Saatgut mit mindestens einer Verbindung der Formel (I) oder einer von Formel (I) abgeleiteten Formel wie hierin beschrieben behandelt wird.

Noch ein weitere Aspekt betrifft die Verwendung von Verbindungen der Formel (I) oder einer von Formel (I) abgeleiteten Formel wie hierin beschrieben oder von einem insektiziden Mittel wie hierin beschrieben zum Bekämpfen von Schädlingen.

Ein weiterer Aspekt betrifft die Verwendung von Verbindungen der Formel (I) oder einer von Formel (I) abgeleiteten Formel wie hierin beschrieben in der Vektorkontrolle.

Noch ein weiterer Aspekt betrifft Saatgut, bei dem eine Verbindung der Formel (I) oder einer von Formel (I) abgeleiteten Formel wie hierin beschrieben als Bestandteil einer Umhüllung oder als weitere Schicht oder weitere Schichten zusätzlich zu einer Umhüllung auf das Saatgut aufgebracht ist.

Entsprechend betrifft ein weiterer Aspekt ein Verfahren zum Aufbringen einer Umhüllung umfassend mindestens eine Verbindungen der Formel (I) oder einer von Formel (I) abgeleiteten Formel wie hierin beschrieben oder zum Aufbringen einer Verbindungen der Formel (I) oder einer von Formel (I) abgeleiteten Formel wie hierin beschrieben, die als Schicht oder weitere Schichten zusätzlich zu einer Umhüllung auf Saatgut aufgebracht wird, umfassend die Schritte, a) mischen von Samen mit einem Überzugsmaterial bestehend aus oder umfassend eine Verbindungen der Formel (I) oder einer von Formel (I) abgeleiteten Formel wie hierin beschrieben, b) anreichern der erhaltenen übergezogenen Samenmasse, c) trocknen der erhaltenen angereicherten Samenmasse, d) enthalten (dis- oder entagglomerieren) der erhaltenen getrockneten Samenmasse.

Die hier beschriebenen Verbindungen der Formel (I) oder einer von Formel (I) abgeleiteten Formel können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen können auch als Metallkomplexe vorliegen.

### Definitionen

Der Fachmann ist sich bewusst, dass, wenn nicht ausdrücklich angegeben, die Ausdrücke "ein", "eine" oder "eines" wie in dieser Anmeldung genutztje nach Situation "ein/eine/eines (1)", "ein/eine/eines (1) oder mehr" oder "mindestens ein/eine/eines (1)" bedeuten kann.

Strukturen mit einer variablen Anzahl an möglichen Kohlenstoffatomen (C-Atomen) können in der vorliegenden Anmeldung als C_{untere Grenze C-Atome}-C_{obere Grenze C-Atome}-Strukturen (C_{uG}-C_{oG}-Strukturen) bezeichnet werden, umso näher bestimmt zu werden. Beispiel: eine Alkylgruppe kann aus 3 bis 10 C-Atomen bestehen und entspricht dann C₃-C₁₀-Alkyl. Ringstrukturen aus C-Atomen und Heteroatomen können als "uG bis oG-gliedrige" Strukturen bezeichnet werden. Ein Beispiel einer 6-gliedrigen Ringstruktur ist Toluol (eine 6-gliedriges Ringstruktur, die mit einer Methylgruppe substituiert ist).

Dem Fachmann ist klar, dass in dieser Anmeldung genannte Beispiele nicht als beschränkend anzusehen sind sondern lediglich einige Ausführungsformen näher beschreiben.

Bei zusammengesetzen Substituenten (z. B. Hydroxyalkyl oder Alkoxy oder Alkylcarbonyl) ist der zweitgenannte Substituente jeweils mit der Grundstruktur Verbunden (z. B. Hydroxymethyl: -CH₂-OH oder Methoxy: -O-CH₃ oder Methylcarbonyl: -C(=O)-CH₃).

Bei den in den vorstehenden Formeln angegebenen Definitionen der Symbole wurden Sammelbegriffe verwendet, die allgemein repräsentativ für die folgenden Substituenten stehen:

Halogen bezieht sich auf die Elemente der 7. Hauptgruppe, bevorzugt Fluor, Chlor, Brom und Iod, mehr bevorzugt Fluor, Chlor und Brom.

Erfindungsgemäß steht "Alkyl" - in Alleinstellung oder als Bestandteil einer chemischen Gruppe - für geradkettige oder verzweigte Kohlenwasserstoffe, vorzugsweise mit 1 bis 6 Kohlenstoffatomen, wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt sind Alkyle mit 1 bis 4 Kohlenstoffatomen, wie unter anderem Methyl, Ethyl, Ethyl, n-Propyl, Isopropyl, n-Butyl, Isobutyl, s-Butyl oder t-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt sind Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt sind Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt sind Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Methylcyclopropyl, Ethylcyclopropyl, Isopropylcyclobutyl, 3-Methylcyclopentyl und 4-Methyl-cyclohexyl. Ferner bevorzugt sind Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt sind Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt sind Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes -O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt sind Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Alkylsulfanyl" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt sind Alkylsulfanylgruppen mit 1 bis 4 Kohlenstoffatomen. Die erfindungsgemäßen Alkylsulfanylgruppen können mit einem oder mehreren, gleichen oder verschiedenen Resten substituiert sein.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt sind Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt sind Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt sind Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt sind Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Erfindungsgemäß steht *"N,N*-Dialkylamino-carbonyl" für geradkettiges oder verzweigtes *N,N-*Dialkylaminocarbonyl mit vorzugsweise 1 bis 6 Kohlenstoffatomen oder 1 bis 4 Kohlenstoffatomen im Alkylteil, wie beispielsweise N,N-Dimethylamino-carbonyl, N.N-Diethylamino-carbonyl, N,N-Di(n-propylamino)-carbonyl, N,N-Di-(isopropylamino)-carbonyl und N,N-Di-(s-butylamino)-carbonyl. Die erfindungsgemäßen *N*,*N*-Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im C₁-C₄-Alkyl- und/oder C₆-C₁₄-Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2. 1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.
Der Begriff "(gegebenenfalls) substituierte" Gruppen/Substituenten, wie ein substituierter Alkyl-, Alkenyl-, Alkinyl-, Alkoxy-, Alkylsulfanyl-, Alkylsulfinyl-, Alkylsulfonyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise ein (1) Substituent oder mehrere Substituenten, vorzugsweise 1, 2, 3, 4, 5, 6, oder 7, ausgewählt sind aus einer Gruppe bestehend aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N*-Di-C₁-C₄-alkylamino, *N-*C₁*-*C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst ind, C₁-C₄-Alkylsulfonyl, N-Mono-C₁-C₄-alkyl-aminosulfonyl, *N*,*N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N*,*N*-Di-C₁-C₄-alkyl-amino-carbonyl, *N*-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundene Substituenten wie C₁-C₄-Alkyliden (z. B. Methyliden oder Ethyliden), eine Oxogruppe, eine Iminogruppe sowie einer substituierten Iminogruppe. Besonders bevorzugte substituierte Gruppen sind halogenierte Gruppen. Diese können eine oder mehrere Halogensubstituenten besitzen, jedoch besitzen diese Gruppen noch mindestens eine C-H Bindung, oder perhalogieniert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen oder mehreren der Substituenten jeweils unabhängig voneinander ausgewählt aus Halogen, Hydroxy, Amino, Nitro, Cyano, Isocyano, Azido, Acylamino, einer Oxogruppe und einer Iminogruppe. Vorzugsweise werden vom Begriff "(gegebenenfalls) substituierter" Gruppe nur ein oder zwei Substitutentenebenen umfasst.

Die erfindungsgemäßen mit Halogen substituierten chemischen Gruppen bzw. halogenierte Gruppen (wie z. B. Alkyl oder Alkoxy) sind einfach oder mehrfach bis zur maximal möglichen Substituentenzahl mit Halogen substituiert. Solche Gruppen werden auch als Halogruppen bezeichnet (wie, Z. B. Haloalkyl). Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor oder Cl. Insbesondere sind mit Halogen substituierte Gruppen Monohalocycloalkyl wie 1-Fluor-cyclopropyl, 2-Fluor-cyclopropyl oder 1-Fluor-cyclobutyl, Monohaloalkyl wie 2-Chlor-ethyl, 2-Fluor-ethyl, 1-Chlor-ethyl, 1-Fluor-ethyl, Chlormethyl, oder Fluormethyl; Perhaloalkyl wie Trichlormethyl oder Trifluormethyl oder CF₂CF₃, Polyhaloalkyl wie Difluormethyl, 2-Fluor-2-Chlor-ethyl, Dichlormethyl, 1,1,2,2-Tetraflourethyl, oder 2,2,2-Trifluorethyl. Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl, 3,3,3-Trifluorpropyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl. Weitere Beispiele für mit Halogen substituierten Verbindungen sind Haloalkoxy wie OCF₃, OCHF₂, OCH₂F, OCF₂CF₃, OCH₂CF₃, OCH₂CHF₂ und OCH₂CH₂Cl, Halogenalkylsulfanyle wie Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio, Halogenalkylsulfinyle wie Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl, Halogenalkylsulfinyle wie Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl, Halogenalkylsulfonylgrupen wie Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Bei Resten mit C-Atomen sind solche mit 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise N-Mono- und *N*,*N*-Dialkylamino, (z.B. Methylamino, Ethylamino, *N*,*N*-Dimethylamino, *N,N-*Diethylamino, *N*,*N*-Di-n-propylamino, *N*,*N*-Diisopropylamino oder *N*,*N*-Dibutylamino), *N*-Mono- oder N,N-Dialkoxyalkylaminogruppen (z.B. *N*-Methoxymethylamino, *N*-Methoxyethylamino, *N,N-Di-*(methoxymethyl)-amino oder *N*,*N*-Di-(methoxyethyl)-amino), *N*-Mono- und *N*,*N*-Diarylamino, wie gegebenenfalls substituierte Aniline, Acylamino, *N*,*N*-diacylamino, *N*-Alkyl-N-arylamino, *N*-Alkyl-*N-*acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, C₁-C₄-Alkyl, C₁-C₄-Alkoxy, C₁-C₄₋Alkoxy-C₁-C₄₋alkoxy , C₁-C₄₋Alkoxy-C₁-C₄₋alkyl, C₁-C₄₋Halogenalkyl, C₁-C₄₋Halogenalkoxy, C₁-C₄-Alkylsulfanyl, C₁-C₄₋Halogenalkylsulfanyl, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl, 4-Heptafluorphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, C₁-C₄-Alkyl, C₁-C₄-Alkoxy, C₁-C₄-Alkoxy-C₁-C₄-alkoxy , C₁-C₄₋Alkoxy-C₁-C₄₋alkyl, C₁-C₄-Halogenalkyl und C₁-C₄-Halogenalkoxy substituiert ist, insbesondere durch einen oder zwei C₁-C₄-Alkylreste substituiert ist.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, C₁-C₄-Alkyl, C₁-C₄-Alkoxy, C₁-C₄-Alkoxy-C₁-C₄-alkoxy , C₁-C₄₋Alkoxy-C₁-C₄₋alkyl, C₁-C₄-Halogenalkyl, C₁-C₄-Halogenalkoxy, Nitro und Oxo substituiert ist, insbesondere ein- oder mehrfach durch Reste aus der Gruppe Halogen, C₁-C₄-Alkyl, C₁-C₄-Alkoxy, C₁-C₄-Halogenalkyl und Oxo, ganz besonders durch einen oder zwei C₁-C₄-Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl,, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäße Verbindungen können in bevorzugten Ausführungsformen vorkommen. Einzelne hierin beschriebene Ausführungsformen können dabei miteinander kombiniert werden. Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung wird unter dem Begriff "Hygiene" die Gesamtheit aller Maßnahmen, Verfahren und Verhaltensweisen verstanden, die das Ziel haben, Erkrankungen - insbesondere Infektionskrankheiten - zu vermeiden und der Gesunderhaltung des Menschen, Tieres und/oder der Umwelt zu dienen und/oder die Sauberkeit zu erhalten. Hierunter fallen erfindungsgemäß insbesondere Maßnahmen zur Reinigung, Desinfektion und Sterilisation beispielsweise von Textilien oder harten Oberflächen, vornehmlich aus Glas, Holz, Beton, Porzellan, Keramik, Kunststoff oder auch aus Metall(en), und deren Reinhaltung von Hygieneschädlingen bzw. deren Fäkalien. Erfindungsgemäß ausgeschlossen sind diesbezüglich wiederum Verfahren zur chirurgischen oder therapeutischen Behandlung des menschlichen oder tierischen Körpers und Diagnostizierverfahren, die am menschlichen oder tierischen Körper vorgenommen werden.

Der Begriff "Hygienesektor" umfasst demnach alle Bereiche, technischen Gebiete und gewerblichen Nutzungen, in denen derartige Maßnahmen, Verfahren und Verhaltensweisen zur Hygiene von Wichtigkeit sind, wie beispielsweise die Hygiene in Küchen, Bäckereien, Flughäfen, Bädern, Schwimmbädern, Einkaufshäusern, Hotels, Krankenhäusern, Stallungen, etc.

Unter dem Begriff "Hygieneschädling" werden folglich ein oder mehrere tierischer(e) Schädling(e) verstanden, deren Vorhandensein im Hygienesektor insbesondere aus gesundheitlichen Gründen problematisch ist. Es ist folglich das vornehmliche Ziel, Hygieneschädlinge bzw. den Kontakt mit ihnen im Hygienesektor zu vermeiden bzw. zu minimieren. Dies kann insbesondere durch die Anwendung eines Schädlingsbekämpfungsmittels erfolgen, wobei das Mittel sowohl prophylaktisch als auch erst bei Befall zur Bekämpfung des Schädlings eingesetzt werden kann. Es ist ebenfalls möglich, Mittel einzusetzen, die bewirken, dass ein Kontakt mit dem Schädling vermieden oder reduziert wird. Als Hygieneschädlinge kommen beispielsweise die nachstehend genannten Organismen in Betracht.

Der Begriff "Hygieneschutz" umfasst demnach alle Handlungen zur Aufrechterhaltung und/oder Verbesserung von derartigen Maßnahmen, Verfahren und Verhaltensweisen zur Hygiene.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z.B. Periplaneta americana, Periplaneta australasiae, Pycnoscelus surinamensis, Supella longipalpa;
aus der Ordnung der Coleoptera z.B. Acalymma vittatum, Acanthoscelides obtectus, Adoretus spp., Aethina tumida, Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus comutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricome, Latheticus oryzae, Lathridius spp., Lema spp., Leptinotarsa decemlineata, Leucoptera spp., z.B. Leucoptera coffeella, Lissorhoptrus oryzophilus, Listronotus (= Hyperodes) spp., Lixus spp., Luperodes spp., Luperomorpha xanthodera, Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oulema spp., z.B. Oulema melanopus, Oulema oryzae, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Dermaptera z.B. Anisolabis maritime, Forficula auricularia, Labidura riparia;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya oder Pegomyia spp., z.B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex, Toxotrypana curvicauda;
aus der Ordnung der Hemiptera z.B. Acizzia acaciaebaileyanae, Acizzia dodonaeae, Acizzia uncatoides, Acrida turrita, Acyrthosiphon spp., z.B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inomata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Capulinia spp., Cameocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lopholeucaspis japonica, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Neomaskellia spp., Nephotettix spp., z.B. Nephotettix cincticeps,, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Perkinsiella spp., Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pulvinaria spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Aelia spp., Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Megacopta cribraria, Miridae, Monalonion atratum, Nezara spp., z.B. Nezara viridula, Nysius spp., Oebalus spp., Pentomidae, Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Camponotus spp., Dolichovespula spp., Diprion spp., z.B. Diprion similis, Hoplocampa spp., z.B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z.B. Vespa crabro, Wasmannia auropunctata, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Kalotermes spp., Microtermes obesi, Nasutitermes spp., Odontotermes spp., Porotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens, Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z.B. Ostrinia nubilalis, Panolis flammea, Pamara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (= Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Chaetanaphothrips leeuweni, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, z.B. Ancylostoma duodenale, Ancylostoma ceylanicum, Acylostoma braziliensis, Angiostrongylus spp., Anisakis spp., Anoplocephala spp., Ascaris spp., Ascaridia spp., Baylisascaris spp., Brugia spp., z. B. Brugia malayi, Brugia timori, Bunostomum spp., Capillaria spp., Chabertia spp., Clonorchis spp., Cooperia spp., Crenosoma spp., Cyathostoma spp., Dicrocoelium spp., Dictyocaulus spp., z. B. Dictyocaulus filaria, Diphyllobothrium spp., z. B. Diphyllobothrium latum, Dipylidium spp., Dirofilaria spp., Dracunculus spp., z. B. Dracunculus medinensis, Echinococcus spp., z.B. Echinococcus granulosus, Echinococcus multilocularis, Echinostoma spp., Enterobius spp., z.B. Enterobius vermicularis, Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., z.B. Hymenolepis nana, Hyostrongylus spp., Litomosoides spp., Loa spp., z.B. Loa Loa, Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, z.B. Onchocerca volvulus, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., z.B. Strongyloides fuelleborni, Strongyloides stercoralis, Strongylus spp., Syngamus spp., Taenia spp., z.B. Taenia saginata, Taenia solium, Teladorsagia spp., Thelazia spp., Toxascaris spp., Toxocara spp., Trichinella spp., z.B. Trichinella spiralis, Trichinella nativa, Trichinella britovi, Trichinella nelsoni, Trichinella pseudopsiralis, Trichobilharzia spp., Trichostrongylus spp., Trichuris spp., z.B. Trichuris trichuria, Uncinaria spp., Wuchereria spp., z.B. Wuchereria bancrofti;

Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella omata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hirschmaniella spp., Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z.B. Paratrichodorus minor, Paratylenchus spp., Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia, z.B. Eimeria spp., bekämpfen.

### Nematoden

Der Begriff "Nematoden" umfasst im vorliegenden Zusammenhang alle Arten des Stammes Nematoda und hierbei insbesondere Arten, die Pflanzen oder Pilze (zum Beispiel Arten der Ordnung Aphelenchida, Meloidogyne, Tylenchida und andere) oder auch Menschen und Tiere (zum Beispiel Arten der Ordnungen Trichinellida, Tylenchida, Rhabditida und Spirurida) parasitieren oder in bzw. an diesen Lebewesen Schädigungen verursachen, sowie andere parasitäre Helminthen.

Ein Nematizid im Pflanzenschutz, wie hier beschrieben, besitzt die Fähigkeit, Nematoden zu kontrollieren.

Der Begriff "Nematoden kontrollieren" bedeutet das Abtöten der Nematoden oder das Verhindern oder Erschweren ihrer Entwicklung bzw. ihres Wachstums oder das Verhindern oder Erschweren ihres Eindringens in oder ihres Saugens am pflanzlichen Gewebe.

Dabei wird die Wirksamkeit der Verbindungen durch einen Vergleich von Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden zwischen einer mit der Verbindung der Formel (I) behandelten Pflanze, Pflanzenteil oder dem behandelten Boden und einer unbehandelten Pflanze, Pflanzenteil oder unbehandelten Boden (100 %) ermittelt. Vorzugsweise wird eine Verringerung um 25-50 % im Vergleich mit einer unbehandelten Pflanze, Pflanzenteil oder unbehandelten Boden, besonders bevorzugt eine Verringerung um 51 - 79 % und ganz besonders bevorzugt das vollständige Abtöten oder die vollständige Verhinderung von Entwicklung und Wachstum der Nematoden durch eine Verringerung um 80 bis 100 % erreicht. Die Kontrolle von Nematoden, wie hier beschreiben, beinhaltet ebenso die Kontrolle der Nematoden-Vermehrung (Entwicklung von Zysten und/oder Eier). Verbindungen der Formel (I) können ebenso verwendet werden, um die Pflanzen oder Tiere gesund zu erhalten und können kurativ, präventiv oder systemisch zur Nematoden-Kontrolle eingesetzt werden.

Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden bestimmt werden.

Die Verwendung einer Verbindung der Formel (I) kann die Pflanze gesund erhalten und beinhaltet ebenso eine Reduktion der von Nematoden hervorgerufenen Schäden sowie eine Erhöhung der Erntemenge.

Der Begriff "Nematoden" bezieht sich im vorliegenden Zusammenhang auf Pflanzennematoden, unter die man alle Nematoden zusammenfasst, die Pflanzen schädigen. Pflanzennematoden umfassen pflanzenparasitäre Nematoden und im Boden lebende Nematoden. Zu den pflanzenparasitären Nematoden zählen Ektoparasiten wie Xiphinema spp., Longidorus spp. und Trichodorus spp.; Halbparasiten wie Tylenchulus spp.; migratorische Endoparasiten wie Pratylenchus spp., Radopholus spp. und Scutellonema spp.; ortsgebundene Parasiten wie Heterodera spp., Globodera spp. und Meloidogyne spp., sowie Stängel- und Blattendoparasiten wie Ditylenchus spp., Aphelenchoides spp. und Hirschmaniella spp.. Besonders schädliche wurzelparasitäre Bodennematoden sind zum Beispiel zystenbildende Nematoden der Gattungen Heterodera oder Globodera, und/oder Wurzelgallennematoden der Gattung Meloidogyne. Schädliche Arten dieser Gattungen sind zum Beispiel Meloidogyne incognita, Heterodera glycines (Sojabohnenzystennematode), Globodera pallida und Globodera rostochiensis (Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den Pflanzennematoden zählen z.B. Aglenchus agricola, Anguina tritici, Aphelenchoides arachidis, Aphelenchoides fragaria und die Stängel- und Blattendoparasiten Aphelenchoides spp., Belonolaimus gracilis, Belonolaimus longicaudatus, Belonolaimus nortoni, Bursaphelenchus cocophilus, Bursaphelenchus eremus, Bursaphelenchus xylophilus und Bursaphelenchus spp., Cacopaurus pestis, Criconemella curvata, Criconemella onoensis, Criconemella omata, Criconemella rusium, Criconemella xenoplax (= Mesocriconema xenoplax) und Criconemella spp.,

Criconemoides ferniae, Criconemoides onoense, Criconemoides omatum und Criconemoides spp., Ditylenchus destructor, Ditylenchus dipsaci, Ditylenchus myceliophagus sowie die Stängel- und Blattendoparasiten Ditylenchus spp., Dolichodorus heterocephalus, Globodera pallida (=Heterodera pallida), Globodera rostochiensis (Kartoffelzystennematode), Globodera solanacearum, Globodera tabacum, Globodera virginia und die ortsgebundenen zystenbildenden Parasiten Globodera spp., Helicotylenchus digonicus, Helicotylenchus dihystera, Helicotylenchus erythrine, Helicotylenchus multicinctus, Helicotylenchus nannus, Helicotylenchus pseudorobustus und Helicotylenchus spp., Hemicriconemoides, Hemicycliophora arenaria, Hemicycliophora nudata, Hemicycliophora parvana, Heterodera avenae, Heterodera cruciferae, Heterodera glycines (Sojabohnenzystennematode), Heterodera oryzae, Heterodera schachtii, Heterodera zeae und die ortsgebundenen zystenbildenden Parasiten Heterodera spp., Hirschmaniella gracilis, Hirschmaniella oryzae, Hirschmaniella spinicaudata und die Stängel- und Blattendoparasiten Hirschmaniella spp., Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola und die Ektoparasiten Longidorus spp., Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne fallax, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne minor, Meloidogyne naasi, Meloidogyne paranaensis, Meloidogyne thamesi und die ortsgebundenen Parasiten Meloidogyne spp., Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres und Paratrichodorus spp., Paratylenchus hamatus, Paratylenchus minutus, Paratylenchus projectus und Paratylenchus spp., Pratylenchus agilis, Pratylenchus allem, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans,Pratylenchus pratensis, Pratylenchus scribneri, Pratylenchus teres, Pratylenchus thornei, Pratylenchus vulnus, Pratylenchus zeae und die migratorischen Endoparasiten Pratylenchus spp., Pseudohalenchus minutus, Psilenchus magnidens, Psilenchus tumidus, Punctodera chalcoensis, Quinisulcius acutus, Radopholus citrophilus, Radopholus similis, die migratorischen Endoparasiten Radopholus spp., Rotylenchulus borealis, Rotylenchulus parvus, Rotylenchulus reniformis und Rotylenchulus spp., Rotylenchus laurentinus, Rotylenchus macrodoratus, Rotylenchus robustus, Rotylenchus uniformis und Rotylenchus spp., Scutellonema brachyurum, Scutellonema bradys, Scutellonema clathricaudatum und die migratorischen Endoparasiten Scutellonema spp., Subanguina radiciola, Tetylenchus nicotianae, Trichodorus cylindricus, Trichodorus minor, Trichodorus primitivus, Trichodorus proximus, Trichodorus similis, Trichodorus sparsus und die Ektoparasiten Trichodorus spp., Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp., Tylenchulus semipenetrans und die Semiparasiten Tylenchulus spp., Xiphinema americanum, Xiphinema brevicolle, Xiphinema dimorphicaudatum, Xiphinema index und die Ektoparasiten Xiphinema spp.

Zu den Nematoden, zu deren Bekämpfung eine Verbindung der Formel (I) eingesetzt werden kann, zählen Nematoden der Gattung Meloidogyne wie der Southern Root-Knot Nematode (Meloidogyne incognita), der Javanese Root-Knot Nematode (Meloidogyne javanica, der Northern Root-Knot Nematode (Meloidogyne hapla) und der Peanut Root-Knot Nematode (Meloidogyne arenaria); Nematoden der Gattung Ditylenchus wie das Kartoffelkrätzeälchen (Ditylenchus destructor) und das Stock- und Stängelälchen (Ditylenchus dipsaci); Nematoden der Gattung Pratylenchus wie der Cob Root-Lesion Nematode (Pratylenchus penetrans), der Chrysanthemum Root-Lesion Nematode (Pratylenchus fallax), der Kaffeewurzelnematode (Pratylenchus coffeae), der Teewurzelnematode (Pratylenchus loosi) und der Walnut Root-Lesion Nematode (Pratylenchus vulnus); Nematoden der Gattung Globodera wie das Goldfarbene Kartoffelzystenälchen (Globodera rostochiensis) und das Weiße Kartoffelzystenälchen (Globodera pallida); Nematoden der Gattung Heterodera wie der Sojabohnenzystennematode (Heterodera glycines) und das Rübenzystenälchen (Heterodera schachtii); Nematoden der Gattung Aphelenchoides wie der Rice White-tip Nematode (Aphelenchoides besseyi), das Chrysanthemenälchen (Aphelenchoides ritzemabosi) und das Erdbeerälchen (Aphelenchoides fragariae); Nematoden der Gattung Aphelenchus wie der fungivore Nematode (Aphelenchus avenae); Nematoden der Gattung Radopholus, wie der Burrowing-Nematode (Radopholus similis); Nematoden der Gattung Tylenchulus wie der Orangenwurzelnematode (Tylenchulus semipenetrans); Nematoden der Gattung Rotylenchulus wie der reniforme Nematode (Rotylenchulus reniformis); in Bäumen lebende Nematoden, wie der Kiefernholznematode (Bursaphelenchus xylophilus) und der Red Ring Nematode (Bursaphelenchus cocophilus) und dergleichen.

Zu den Pflanzen, zu deren Schutz eine Verbindung der Formel (I) verwendet werden kann, zählen Pflanzen wie Getreide (zum Beispiel Reis, Gerste, Weizen, Roggen, Hafer, Mais, und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Röhrenlauch, Pfeffer und dergleichen), Hackfrüchte (Karotte, Kartoffel, Süßkartoffel, Rettich, Lotuswurzel, Steckrübe und dergleichen), Pflanzen für industrielle Rohstoffe (Baumwolle, Hanf, Papiermaulbeere, Mitsumata, Raps, Rübe, Hopfen, Zuckerrohr, Zuckerrübe, Olive, Gummi, Palmen, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumenpflanzen (Chrysantheme, Rose, Orchidee und dergleichen) erwähnt werden.

Die Verbindungen der Formel (I) eignen sich besonders für die Bekämpfung von Nematoden des Kaffees, insbesondere von Pratylenchus brachyurus, Pratylenchus coffeae, Meloidogyne exigua, Meloidogyne incognita, Meloidogyne coffeicola, Helicotylenchus spp. sowie aus Meloidogyne paranaensis, Rotylenchus spp., Xiphinema spp., Tylenchorhynchus spp. und Scutellonema spp..

Die Verbindungen der Formel (I) eignen sich besonders für die Bekämpfung von Nematoden der Kartoffel, insbesondere von Pratylenchus brachyurus, Pratylenchus pratensis, Pratylenchus scribneri, Pratylenchus penetrans, Pratylenchus coffeae, Ditylenchus dipsaci sowie aus Pratylenchus allem, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne fallax, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoides fragariae und Meloinema spp.

Die Verbindungen der Formel (I) eignen sich besonders für die Bekämpfung von Nematoden der Tomate, insbesondere von Meloidogyne arenaria, Meloidogyne hapla, Meloidogyne javanica, Meloidogyne incognita, Pratylenchus penetrans und aus Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus und Rotylenchulus reniformis.

Die Verbindungen der Formel (I) eignen sich besonders für die Bekämpfung von Nematoden von Gurkengewächsen, insbesondere von Meloidogyne arenaria, Meloidogyne hapla, Meloidogyne javanica, Meloidogyne incognita, Rotylenchulus reniformis und Pratylenchus thornei.

Die Verbindungen der Formel (I) eignen sich besonders für die Bekämpfung von Nematoden der Baumwolle, insbesondere von Belonolaimus longicaudatus, Meloidogyne incognita, Hoplolaimus columbus, Hoplolaimus galeatus und Rotylenchulus reniformis.

Die Verbindungen der Formel (I) eignen sich besonders für die Bekämpfung von Nematoden des Maises, insbesondere von Belonolaimus longicaudatus, Paratrichodorus minor und aus Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella omata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum und Subanguina radiciola.

Die Verbindungen der Formel (I) eignen sich besonders für die Bekämpfung von Nematoden der Sojabohne, insbesondere vonPratylenchus brachyurus, Pratylenchus pratensis, Pratylenchus penetrans, Pratylenchus scribneri, Belonolaimus longicaudatus, Heterodera glycines, Hoplolaimus columbus und aus Pratylenchus coffeae, Pratylenchus hexincisus, Pratylenchus neglectus, Pratylenchus crenatus, Pratylenchus allem, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus und Rotylenchulus reniformis.

Die Verbindungen der Formel (I) eignen sich besonders für die Bekämpfung von Nematoden des Tabaks, insbesondere von Meloidogyne incognita, Meloidogyne javanica und aus Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp. und Tetylenchus nicotianae.

Die Verbindungen der Formel (I) eignen sich besonders für die Bekämpfung von Nematoden von Zitrusgewächsen, insbesondere von Pratylenchus coffeae und aus Pratylenchus brachyurus, Pratylenchus vulnus, Belonolaimus longicaudatus, Paratrichodorus minor, Paratrichodorus porosus, Trichodorus , Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, Radopholus similis bzw. Radopholus citrophilus, Hemicycliophora arenaria, Hemicycliophora nudata und Tylenchulus semipenetrans .

Die Verbindungen der Formel (I) eignen sich besonders für die Bekämpfung von Nematoden der Banane, insbesondere von Pratylenchus coffeae, Radopholus similis und aus Pratylenchus giibbicaudatus, Pratylenchus loosi, Meloidogyne spp., Helicotylenchus multicinctus, Helicotylenchus dihystera und Rotylenchulus spp..

Die Verbindungen der Formel (I) eignen sich besonders für die Bekämpfung von Nematoden der Ananas, insbesondere von Pratylenchus zeae, Pratylenchus pratensis, Pratylenchus brachyurus, Pratylenchus goodeyi., Meloidogyne spp., Rotylenchulus reniformis und aus Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense und Criconemoides omatum .

Die Verbindungen der Formel (I) eignen sich besonders für die Bekämpfung von Nematoden von Trauben, insbesondere von Pratylenchus vulnus, Meloidogyne arenaria, Meloidogyne incognita, Meloidogyne javanica, Xiphinema americanum, Xiphinema index und aus Pratylenchus pratensis, Pratylenchus scribneri, Pratylenchus neglectus, Pratylenchus brachyurus, Pratylenchus thornei und Tylenchulus semipenetrans.

Die Verbindungen der Formel (I) eignen sich besonders für die Bekämpfung von Nematoden von Baumkulturen - Kernobst, insbesondere von Pratylenchus penetrans und aus Pratylenchus vulnus, Longidorus elongatus, Meloidogyne incognita und Meloidogyne hapla.

Die Verbindungen der Formel (I) eignen sich besonders für die Bekämpfung von Nematoden von Baumkulturen - Steinfrüchten, insbesondere von Pratylenchus penetrans, Pratylenchus vulnus, Meloidogyne arenaria, Meloidogyne hapla, Meloidogyne javanica, Meloidogyne incognita, Criconemella xenoplax und aus Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum und Hoplolaimus galeatus.

Die Verbindungen der Formel (I) eignen sich besonders für die Bekämpfung von Nematoden in Baumkulturen, Zuckerrohr und Reis , insbesondere von Trichodorus spp., Criconemella spp. und aus Pratylenchus spp. , Paratrichodorus spp., Meloidogyne spp. , Helicotylenchus spp., Tylenchorhynchus spp., Aphelenchoides spp. Heterodera spp, Xiphinema spp. und Cacopaurus pestis.

Ebenso bezieht sich der Begriff "Nematoden" im vorliegenden Zusammenhang auf Nematoden, die Menschen oder Tiere schädigen.

Spezifische Nematodenarten, die für den Menschen oder für Tiere schädlich sind, sind:
Trichinellida, zum Beispiel: Trichuris spp., Capillaria spp., Paracapillaria spp., Eucoleus spp., Trichomosoides spp., Trichinella spp.

Aus der Ordnung der Tylenchida zum Beispiel: Micronema spp., Strongyloides spp.

Aus der Ordnung der Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.

Aus der Odnung der Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;

Viele bekannte Nematizide wirken gleichsam gegen andere parasitäre Helminthen und werden daher für die Bekämpfung von human- und tierparasitären Würmern, die nicht unbedingt zu der Gruppe Nematoda gehören, verwendet. Die vorliegende Erfindung bezieht sich auch auf die Verwendung der Verbindungen der Formel (I) als anthelmintische Arzneimittel. Zu den pathogenen endoparasitären Helminthen zählen Platyhelmintha (z.B. Monogenea, Cestodes und Trematodes), Acanthocephala und Pentastoma. Die folgenden Helminthen sind als bevorzugt zu erwähnen:
Monogenea: z.B.: Gyrodactylus spp., Dactylogyrus spp., Polystoma spp.

Cestodes: aus der Ordnung der Pseudophyllidea zum Beispiel: Diphyllobothrium spp., Spirometra spp., Schistocephalus spp., Ligula spp., Bothridium spp., Diplogonoporus spp.

Aus der Ordnung der Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse der Digenea zum Beispiel: Diplostomum spp., Posthodiplostomum spp., Schistosoma spp., Trichobilharzia spp., Omithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp.

Acanthocephala: aus der Ordnung der Oligacanthorhynchida z.B.: Macracanthorhynchus spp., Prosthenorchis spp.; aus der Ordnung der Polymorphida zum Beispiel: Filicollis spp.; aus der Ordnung der Moniliformida zum Beispiel: Moniliformis spp.,

Aus der Ordnung der Echinorhynchida zum Beispiel Acanthocephalus spp., Echinorhynchus spp., Leptorhynchoides spp.

Pentastoma: aus der Ordnung der Porocephalida zum Beispiel Linguatula spp.

Auf dem Gebiet der Tiermedizin und in der Tierhaltung erfolgt die Verabreichung der Verbindungen der Formel (I) auf bekannte Weise direkt oder enteral, parenteral, dermal oder nasal in Form von geeigneten Anwendungsformen. Die Verabreichung kann prophylaktisch oder therapeutisch erfolgen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Be-tracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamineethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Momfluorothrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.
Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Broflanilide, Bromopropylate, Chinomethionat, Cryolite, Cyclaniliprole, Cycloxaprid, Cyhalodiamide Dicloromezotiaz, Dicofol, Diflovidazin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, Lotilaner, Meperfluthrin, Paichongding, Pyflubumide, Pyridalyl, Pyrifluquinazon, Pyriminostrobin, Sarolaner, Tetramethylfluthrin, Tetraniliprole, Tetrachlorantraniliprole, Tioxazafen, Thiofluoximate, Triflumezopyrim und Iodmethan; desweiteren Präparate auf Basis von Bacillus firmus (I-1582, BioNeem, Votivo), sowie folgende bekannte wirksame Verbindungen: 1- {2-Fluor-4-methyl-5-[(2,2,2-trifluorethyl)sulfinyl]phenyl} -3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon(bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl} amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy} propoxy)-2-methoxy-6-(trifluormethyl)pyrimidin (bekannt aus CN 1 0 133 7940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, Butyl-[2-(2,4-dichlorphenyl)-3-oxo-4-oxaspiro[4.5]dec-1-en-1-yl]-carbonat (bekannt aus CN 102060818), 3E)-3-[1-[(6-Chlor-3-pyridyl)methyl]-2-pyridyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926), 5-Brom-4-chlor-N-[4-chlor-2-methyl-6-(methylcarbamoyl)phenyl]-2-(3-chlor-2-pyridyl)pyrazole-3-carboxamid (bekannt aus CN103232431).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1 -(2,2-Dimethyl-2,3-dihydro-1H-inden-1-yl)-1H-imidazol-5-carbonsäuremethylester, (1.62) N'- {5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl} -2,4-dihydro-3H-1,2,4-triazol-3-thion, (1.67) 1-{[3-(2-Chlorphenyl)-2-(2,4-difluorphenyl)oxiran-2-yl]methyl} - 1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl} - 1H-1,2,4-triazol-5-ylthiocyanat, (1.73) 1-{[rel(2R,3R)-3-(2-Chlorphenyl)-2-(2,4-difluorphenyl)oxiran-2-yl]methyl} -1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1 - { [rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2-[4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl] -1 -(1H-1,2,4-triazol-1 -yl)propan-2-ol, (1.89) (2R)-2-(1-Chlorcyclopropyl)-4-[(1R)-2,2-dichlorcyclopropyl]-1 -(1H-1,2,4-triazol-1 -yl)butan-2-ol, (1.90) (2R)-2-(1 -Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1 -methyl-N-(1,1,3 -trimethyl-2,3 -dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3 -trimethyl-2,3 -dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H-pyrazol-4-carboxamid, (2.46) N-(2',4'-Dichlorbiphenyl-2-yl)-3-(difluormethyl)-1-methyl-1H-pyrazol-4-carboxamid, (2.47) 3-(Difluormethyl)-1-methyl-N-[4'-(trifluormethyl)biphenyl-2-yl]-1H-pyrazol-4-carboxamid, (2.48) N-(2',5'-Difluorbiphenyl-2-yl)-1-methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylacetamid, (3.24) (2E)-2-(Methoxyimino)-N-methyl-2-(2- {[({(1E)-1-[3-(trifluormethyl)phenyl]ethyliden} amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl} acetamid, (3.26) (2E)-2- {2-[({[(1E)-1-(3- {[(E)-1-Fluor-2-phenylvinyl]oxy}phenyl)ethyliden]amino}oxy)methyl]phenyl}-2-(methoxyimino)-N-methylacetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxyacrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1 -(morpholin-4-yl)prop-2-en-1 -on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl} carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothal-isopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1 -yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2-[(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxy} -2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-1-yloxy)phenyl]propanamid, (15.078) N-[(5-Brom-3-chlorpyridin-2-yl)methyl]-2,4-dichlomicotinamid, (15.079) N-[1-(5-Brom-3-chlorpyridin-2-yl)ethyl]-2,4-dichlomicotinamid, (15.080) N-[1-(5-Brom-3-chlorpyridin-2-yl)ethyl]-2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}-carbamidsäurepentylester, (15.088) Phenazin-1-carbonsäure, (15.089) Chinolin-8-ol, (15.090) Chinolin-8-olsulfat (2:1), (15.091) {6-[({[(1-Methyl-1H-tetrazol-5-yl)(phenyl)methylen]amino}-oxy)methyl]pyridin-2-yl}carbamidsäure-tert.-butylester, (15.092) (5-Brom-2-methoxy-4-methylpyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]-amino}oxy)methyl]pyridin-2-yl}carbamidsäurebut-3-in-1-ylester, (15.096) 4-Amino-5-fluorpyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'-{5-Brom-6-[1-(3,5-difluorphenyl)ethoxy]-2-methylpyridin-3-yl}-N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N-[2-Chlor-6-(trifluormethyl)benzyl]-N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, (15.137) 2-{2-[(7,8-Difluor-2-methylchinolin-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimethyl-1H-pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy} -2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimidoformamid, (15.156) N'-(2,5-Dimethyl-4-{[3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.165) 2-[3,5-Bis(difluormethyl)-1H-pyrazol-1-yl]-1-[4-(4-{(SS)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl} -1,3-thiazol-2-yl)piperidin-1 -yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}- 1,3-thiazol-2-yl)piperidin-1-yl]ethanon, (15.169) 2-[3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1 -yloxy)phenyl]-4,5-dihydro-1,2-oxazol-3-yl} -1,3-thiazol-2-yl)piperidin-1 -yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens*, Stamm FZB42 (DSM 231179), oder *Bacillus cereus*, insbesondere *B. cereus* Stamm CNCM I-1562 oder *Bacillus firmus*, Stamm I-1582 (Accession number CNCM I-1582) oder *Bacillus pumilus*, insbesondere Stamm GB34 (Accession No. ATCC 700814) und Stamm QST2808 (Accession No. NRRL B-30087), oder *Bacillus subtilis*, insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis*, insbesondere *B. thuringiensis* subspecies *israelensis* (serotype H-14), Stamm AM65-52 (Accession No. ATCC 1276), oder *B. thuringiensis subsp. aizawai*, insbesondere Stamm ABTS-1857 (SD-1372), oder *B*. *thuringiensis subsp. kurstaki* Stamm HD-1, oder *B. thuringiensis subsp. tenebrionis* Stamm NB 176 (SD-5428), *Pasteuria penetrans*, *Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana*, insbesondere Stamm ATCC 74040, *Coniothyrium minitans*, insbesondere Stamm CON/M/91-8 (Accession No. DSM-9660), *Lecanicillium spp.*, insbesondere Stamm HRO LEC 12, *Lecanicillium lecanii*, (ehemals bekannt als *Verticillium lecanii*), insbesondere Stamm KV01, *Metarhizium anisopliae*, insbesondere Stamm F52 (DSM3884/ ATCC 90448), *Metschnikowia fructicola*, insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus*, insbesondere *P. lilacinus* Stamm 251 (AGAL 89/030550), *Talaromyces flavus*, insbesondere Stamm V117b, *Trichoderma atroviride*, insbesondere Stamm SC1 (Accession Number CBS 122089), *Trichoderma harzianum*, insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als ,Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp*., *Burkholderia spp*., insbesondere *Burkholderia cepacia* (ehemals bekannt als *Pseudomonas cepacia*), *Gigaspora spp*., oder *Gigaspora monosporum*, *Glomus spp*., *Laccaria spp*., *Lactobacillus buchneri*, *Paraglomus spp., Pisolithus tinctorus*, *Pseudomonas spp., Rhizobium spp.*, insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem ^{™} Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika und Chili, Gurken, Melonen, Karotten, Wassermelonen, Zwiebel, Salat, Spinat, Lauch, Bohnen, *Brassica oleracea* (z.B. Kohl) und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien der Pflanzen verstanden werden, beispielsweise Samen, Stecklinge sowie junge (unreife) Pflanzen bis hin zu reifen Pflanzen. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut (geerntete Pflanzen oder Pflanzenteile) sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydomii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Omithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Anthelminthische Mischungspartner

Beispielhaft seien folgende anthelmintische Mischungspartner genannt:

Anthelminthische Wirkstoffe, einschließlich trematicide und cestocide Wirkstoffe:
aus der Klasse der **macrocyclischen Lactone**, z. B.: Abamectin, Doramectin, Emamectin, Eprinomectin, Ivermectin, Milbemycin, Moxidectin, Nemadectin, Selamectin;
aus der Klasse der **Benzimidazole** und **Probenzimidazole**, z. B.: Albendazol, Albendazol- Sulfoxid, Cambendazol, Cyclobendazol, Febantel, Fenbendazol, Flubendazol, Mebendazol, Netobimin, Oxfendazol, Oxibendazol, Parbendazol, Thiabendazol, Thiophanat, Triclabendazol;
aus der Klasse der **Cyclooctadepsipeptide**, z. B.: Emodepsid, PF1022;
aus der Klasse der **Aminoacetonitril-Derivate**, z. B.: Monepantel;
aus der Klasse der **Tetrahydropyrimidine**, z. B.: Morantel, Pyrantel, Oxantel;
aus der Klasse der **Imidazothiazole**, z. B.: Butamisol, Levamisol, Tetramisol;
aus der Klasse der **Salicylanilide**, z. B.: Bromoxanid, Brotianid, Clioxanid, Closantel, Niclosamid, Oxyclozanid, Rafoxanid, Tribromsalan;
aus der Klasse der **Paraherquamide**, z. B.: Derquantel, Paraherquamid;
aus der Klasse der **Aminophenylamidine**, z. B.: Amidantel, deacyliertes Amidantel (dAMD), Tribendimidin;
aus der Klasse der **Organophosphate**, z. B.: Coumaphos, Crufomat, Dichlorvos, Haloxon, Naphthalofos, Trichlorfon;
aus der Klasse der **substituierten Phenole**, z. B.: Bithionol, Disophenol, Hexachlorophen, Niclofolan, Meniclopholan, Nitroxynil;
aus der Klasse der **Piperazinone**, z. B.: Praziquantel, Epsiprantel;
aus anderen diversen Klassen, z. B.: Amoscanat, Bephenium, Bunamidin, Clonazepam, Clorsulon, Diamfenetid, Dichlorophen, Diethylcarbamazin, Emetin, Hetolin, Hycanthon, Lucanthon, Miracil, Mirasan, Niclosamid, Niridazol, Nitroxynil, Nitroscanat, Oltipraz, Omphalotin, Oxamniquin, Paromomycin, Piperazin, Resorantel.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenzbrechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere

Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Ein weiterer Aspekt der Erfindung ist die Verwendung einer Verbindung der Formel (I) als Herbizid.

### Darstellungsverfahren

Die erfindungsgemäßen Verbindungen können nach üblichen, dem Fachmann bekannten Methoden hergestellt werden.

Im Reaktionsschema 1 ist ein allgemeines Darstellungsverfahren für die erfindungsgemäßen Verbindungen (**Ia**) und (**Ib**) abgebildet.

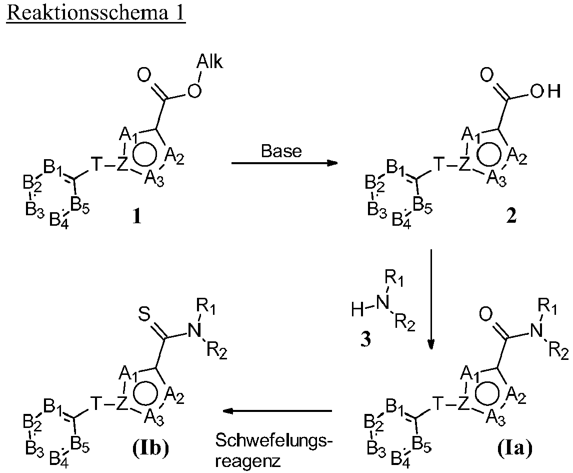

Die Reste A₁, A₂, A₃, Z, T, B₁, B₂, B₃, B₄, B₅, R₁, und R₂ haben die oben beschriebenen Bedeutungen. Alk steht für ein C₁-C₄-Alkyl,

Durch Aktivierung von Intermediat **2** und anschließender Umsetzung der aktivierten Zwischenstufe mit Aminen der allgemeinen Struktur **3** können erfindungsgemäße Verbindungen der allgemeinen Struktur (**Ia**) erhalten werden. Für diesen Amidierungsschritt sind zahlreiche Reaktionsbedingungen beschrieben worden. Eine Zusammenfassung findet sich in Houben-Weyl, Methoden der Organischen Chemie, Band E5 (Georg Thieme Verlag Stuttgart), S. 934. Einige dieser Reaktionen verlaufen über intermediäre Carbonsäurechloride, die isoliert oder in-situ erzeugt eingesetzt werden können. Die Amine der allgemeinen Struktur **3** oder ihre Salze sind kommerziell erhältlich oder nach dem Fachmann bekannten Verfahren darstellbar.

Die Amidfunktion der erfindungsgemäßen Verbindungen der allgemeinen Struktur (**Ia**) kann durch geeignete Schwefelungsreagenzien, z. B. Lawesson's Reagenz oder Phosphor(V)-sulfid in eine Thioamidfunktion überführt werden, wodurch erfindungsgemäße Verbindungen der allgemeinen Struktur (**Ib**) entstehen (siehe z.B. WO 2004/018439).

Carbonsäuren der allgemeinen Struktur **2** können aus Carbonsäureestern der allgemeinen Struktur **1** nach literaturbekannten Methoden mittels Hydrolyse durch geeignete Basen, wie z. B. wässriger Lithiumhydroxid- oder Natriumhydroxid-Lösung, in geeigneten Lösungs- bzw. Verdünnungsmitteln, wie z. B. Dioxan oder THF erhalten werden.

Im Reaktionsschema 1a ist ein allgemeines Darstellungsverfahren für Intermediate der allgemeinen Struktur **1c** abgebildet.

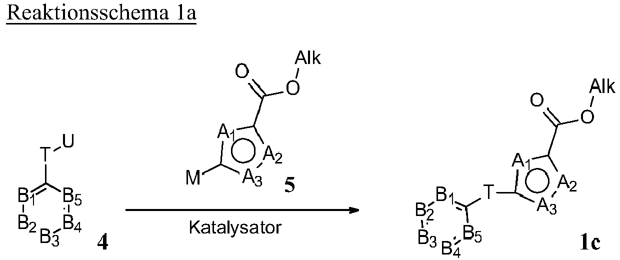

Die Reste A₁, A₂, A₃, B₁, B₂, B₃, B₄, B₅, R₁, und R₂ haben die oben beschriebenen Bedeutungen. T steht für T₁, T₂ oder T₃. Alk steht für ein C₁-C₄-Alkyl. U steht für Brom oder Iod, wenn M für eine Boronsäure, einen Boronsäureester oder ein Zinkhalogenid steht. U steht für eine Boronsäure, einen Boronsäureester oder ein Zinkhalogenid wenn M für Brom, Iod oder Triflat steht.

Die Verbindungen der allgemeinen Struktur **1c** können mittels Palladium katalysierter Reaktionen aus den Reaktionspartnern **4** und **5** hergestellt werden (siehe z.B. WO 2011/149874; WO 2014/152115; WO 2010/093885; Organic & biomolecular chemistry (2016), 14, S. 2352). Die Verbindungen der allgemeinen Struktur **5** sind entweder kommerziell erhältlich oder können nach dem Fachmann bekannten Verfahren hergestellt werden (siehe z.B. WO 2009/106441; WO 2012/038743; Angewandte Chemie, International Edition (2013), 52, S. 7818). Die Verbindungen der allgemeinen Struktur **4** lassen sich nach literaturbekannten Verfahren herstellen (siehe z.B. WO 2016/174052; Organic & biomolecular chemistry (2016), 14, S. 2352).

Im Reaktionsschema 1b ist ein allgemeines Darstellungsverfahren für Intermediate der allgemeinen Struktur **1d** abgebildet.

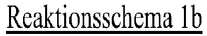

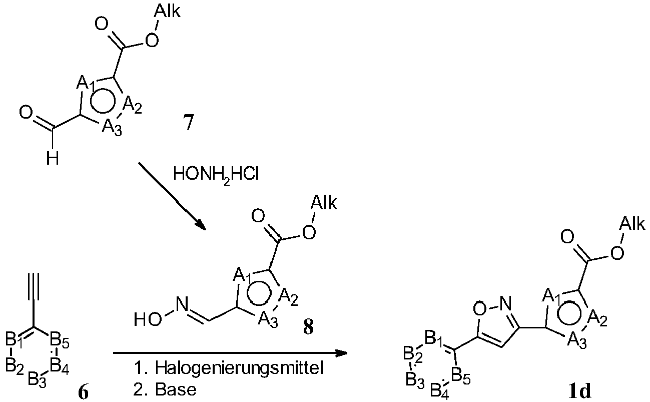

Die Reste A₁, A₂, A₃, B₁, B₂, B₃, B₄ und B₅ haben die oben beschriebenen Bedeutungen. Alk steht für ein C₁-C₄-Alkyl,

Intermediate der allgemeinen Struktur **1d** können analog zu literaturbekannten Methoden aus Intermediaten der allgemeinen Struktur **8** und **6** erhalten werden. Hierbei werden die Oxime **8** zunächst mit geeigneten Halogenierungsmitteln, z.B. *N*-Halosuccinimiden umgesetzt und anschließend mit Acetylenen der allgemeinen Struktur **6** in Gegenwart einer geeigneten Base, z.B. Triethylamin zur Reaktion gebracht (siehe z.B. European Journal of Medicinal Chemistry 2014, 82, 214-224; WO 2016/174052).

Oxime der allgemeinen Struktur **8** sind bekannt oder können analog literaturbekannter Methoden aus Aldehyden der allgemeinen Struktur **7** hergestellt werden (z.B. Bioorganic & Medicinal Chemistry 2012, 20, 5763-5773; J. Chem. Soc. Perk. Trans. 1 1980, 4, 1029-1037). Aldehyde der allgemeinen Struktur **7** sind kommerziell erhältlich oder können nach dem Fachmann bekannten Verfahren hergestellt werden. Alkyne der allgemeinen Struktur **6** sind bekannt oder können analog literaturbekannter Methoden hergestellt werden werden (siehe z.B. WO 2016/174052).

Im Reaktionsschema 1c ist ein allgemeines Darstellungsverfahren für Intermediate der allgemeinen Struktur **1e** abgebildet.

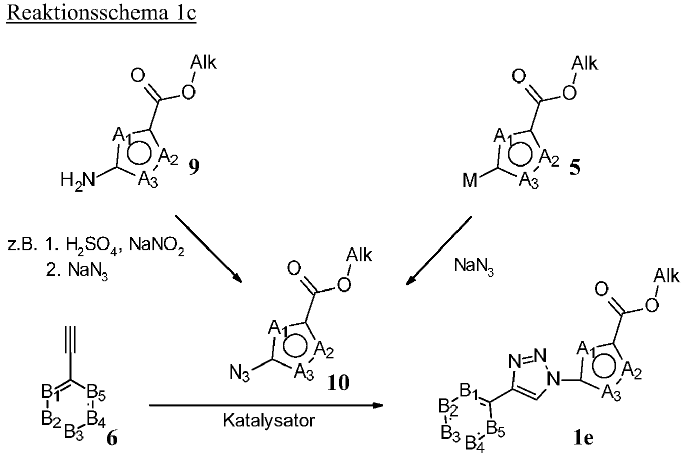

Die Reste A₁, A₂, A₃, B₁, B₂, B₃, B₄ und B₅ haben die oben beschriebenen Bedeutungen. Alk steht für ein C₁-C₄-Alkyl. M steht für Fluor, Chlor, Brom oder Iod.

Intermediate der allgememeinen Struktur **1e** können durch Cu-katalysierte (siehe z.B. WO 2008/074835) Umsetzung von Aziden der allgemeinen Struktur **10** und Acetylenen der allgemeinen Struktur **6** gewonnen werden.

Azide der allgemeinen Struktur **10** können in Analogie zu aus der Literatur bekannten Verfahren ausgehend von Aminen der allgemeinen Struktur **9** erhalten werden (z.B. Tetrahedron Lett. 2013, 54, 1294-1297; Tetrahedron 2009, 65, 2678-2683). Amine der allgemeinen Struktur **9** sind kommerziell erhältlich oder können nach dem Fachmann bekannten Verfahren hergestellt werden.

Alternativ können Azide der allgemeinen Struktur **10** ausgehend von Halogeniden der allgemeinen Struktur **5** (in denen M für Fluor, Chlor, Brom oder Iod steht) durch nukleophile Substitution erhalten werden. Die Reaktion mit Natriumazid verläuft entweder thermisch aktiviert (siehe z.B. WO 2010/121675 oder WO 2012/107434) oder unter Cu-Katalyse (siehe z.B. WO 2014/114532). Die Reaktion unter Cu-Katalyse kann in Gegenwart von Alkynen der allgemeinen Struktur **6** stattfinden, so dass die Umsetzung zu den Intermediaten der allgemeinen Struktur **1c** in einem Eintopfverfahren erfolgt (siehe z.B. Beilstein J. Org. Chem. 2012, 8, 683-692).

Im Reaktionsschema 1d ist ein allgemeines Darstellungsverfahren für Intermediate der allgemeinen Struktur **1f** abgebildet.

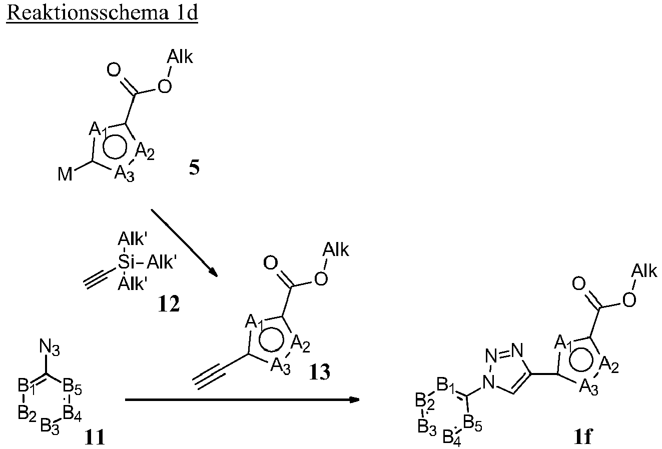

Die Reste A₁, A₂, A₃, B₁, B₂, B₃, B₄ und B₅ haben die oben beschriebenen Bedeutungen. Alk steht für ein C₁-C₄-Alkyl. Alk` steht für ein gebenenfalls substituiertes C₁-C₆-Alkyl. M steht für Brom oder Iod.

Intermediate der allgemeinen Struktur **1f** können durch Cu-katalysierte Umsetzung von Aziden der allgemeinen Struktur **11** und Acetylenen der allgemeinen Struktur **13** gewonnen werden (siehe z.B. Journal of Combinatorial Chemistry 2009, 11, 947; Journal of Medicinal Chemistry 2012, 55, 5642).

Azide der allgemeinen Struktur **11** sind bekannt oder können analog literaturbekannter Methoden hergestellt werden (siehe z.B. WO2016/008830). Alkyne der allgemeinen Struktur **13** sind bekannt oder können in einer zweistufigen Sequenz durch Übergangsmetallkatalysierte Reaktion von Halogeniden der allgemeinen Struktur **5** mit Trialkylsilylacetylenen der allgemeinen Struktur **12** gefolgt von der Abspaltung der Silylgruppe erhalten werden (siehe z.B. WO 2016/055947).

Im Reaktionsschema 1e ist ein Darstellungsverfahren für Intermediate der allgemeinen Struktur **1g** - **1i** abgebildet.

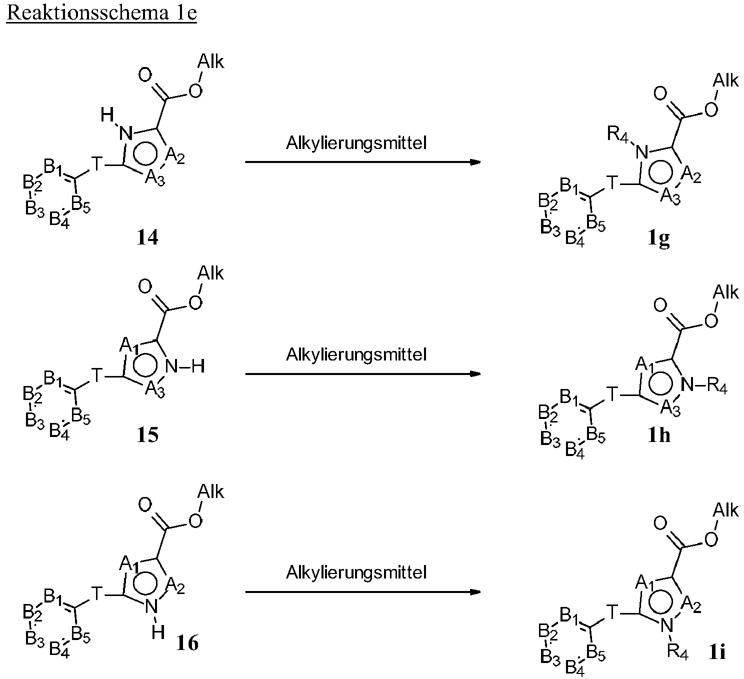

Die Reste B₁, B₂, B₃, B₄, B₅, R₄ und T haben die oben beschriebenen Bedeutungen. Alk steht für ein Ci-C₄-Alkyl. Die Reste A₁, A₂ und A₃ stehen für N oder CR₃.

Intermediate der allgemeinen Struktur **1g** - **1i** können durch Deprotonierung von Intermediaten der allgemeinen Struktur **14** - **16** durch eine geeignete Base und anschließender Reaktion mit Alkylierungsmitteln erhalten werden. Als Alkylierungsmittel werden z.B. Alkylhalogenide verwendet in Kombination mit Alkalimethalhydriden oder Alkalimetallcarbonaten als Base (siehe z.B. WO 2015/011281 für die Kombination Methyliodid und Natriumhydrid, WO 2012/038904 für die Kombination Kaliumcarbonat und Ethylbromid). Die Intermediate der allgemeinen Struktur **14** - **16** können nach den, in Reaktionsschemata 1a - d gezeigten, Darstellungsverfahren erhalten werden. Dazu können unter anderem auch Verbindungen der allgemeinen Struktur **5, 8 10** oder **13** eingesetzt werden, in denen einer der Substituenten A₁, A₂ oder A₃ für N-(tert-Butoxycarbonyl) steht, [siehe z.B. EP 2594555]. Um die Verbindungen der allgemeinen Struktur **14** - **16** zu erhalten muss die (tert-Butoxycarbonyl)-Gruppe gegebenfalls noch unter sauren Bedinungen abgespalten werden (siehe z.B. WO 2013/092512).

Im Reaktionsschema 2 ist ein Darstellungsverfahren für Intermediate der allgemeinen Struktur **1j, 1k** und erfindungsgemäße Verbindungen, die unter die allgemeine Struktur (**Ia**) fallen, abgebildet.

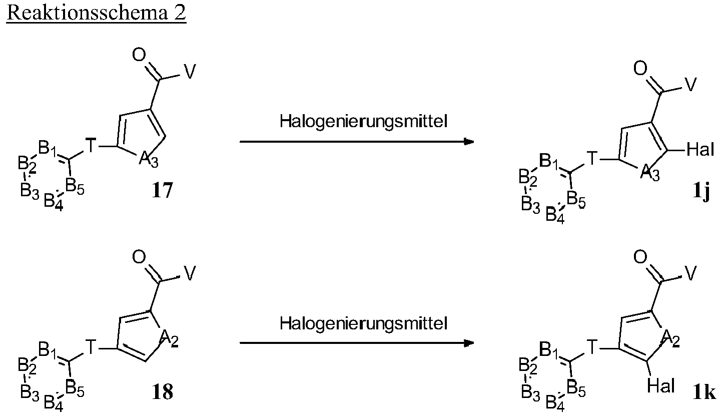

Die Reste B₁, B₂, B₃, B₄, B₅ und T haben die oben beschriebenen Bedeutungen. A₃ und A₂ stehen für S, O oder N-R⁴. Hal steht für Chlor oder Brom. V steht für C₁-C₄-Alkoxy oder NR₁R₂.

Verbindungen der allgemeinen Struktur **1j** und **1k** können durch Reaktion von Intermediaten der allgemeinen Struktur **17** beziehungsweise **18** mit Halogenierungsmitteln erhalten werden. Als Halogenierungsmittel werden z.B. N-Halosuccinimide verwendet (siehe z.B. WO 2009/112845, WO 2008/098105 für Hal = Chlor, WO 2012/137181, WO 2013/079223 für Hal = Brom). Verbindungen der allgemeinen Struktur **1j** und **1k** in denen V für C₁-C₄-Alkoxy steht können entsprechend Reaktionsschema 1 in erfindungsgemäße Verbindungen, die unter die allgemeine Struktur **(I'a)** fallen, überführt werden. Steht V für NR₁R₂ handelt es bei den Verbindungen der allgemeinen Struktur **1j** bis **1k** um erfindungsgemäße Verbindungen der Struktur (**Ia**).

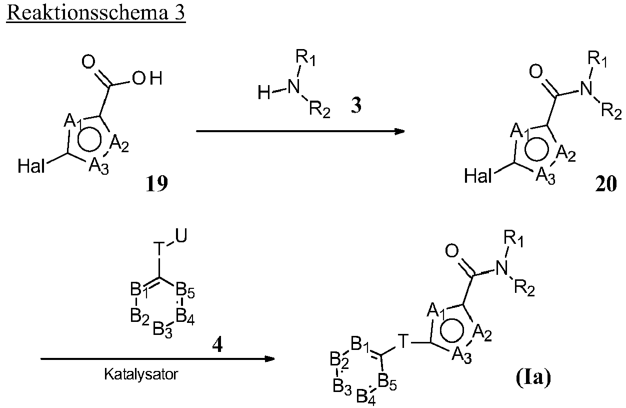

Die Reste A₁, A₂, A₃, B₁, B₂, B₃, B₄, B₅, R₁, und R₂ haben die oben beschriebenen Bedeutungen. T steht für T₁, T₂ oder T₃. Hal steht für Brom oder Iod. U steht für eine Boronsäure, einen Boronsäureester oder ein Zinkhalogenid.

Durch Aktivierung von Carbonsäuren der allgemeinen Struktur **19** und anschließender Umsetzung der aktivierten Zwischenstufe mit Aminen der allgemeinen Struktur **3** können Amide der allgemeinen Struktur **20** erhalten werden. Für diesen Amidierungsschritt sind zahlreiche Reaktionsbedingungen beschrieben worden. Eine Zusammenfassung findet sich in Houben-Weyl, Methoden der Organischen Chemie, Band E5 (Georg Thieme Verlag Stuttgart), S. 934. Einige dieser Reaktionen verlaufen über intermediäre Carbonsäurechloride, die isoliert oder in-situ erzeugt eingesetzt werden können. Die Amine der allgemeinen Struktur **3** oder ihre Salze sind kommerziell erhältlich oder nach dem Fachmann bekannten Verfahren darstellbar. Die Carbonsäuren der allgemeinen Struktur **19** sind kommerziell erhältlich oder nach dem Fachmann bekannten Verfahren darstellbar (siehe z.B. WO2012/033390 für die Synthese von 5-Brom-2-methylthiophen-3-carbonsäure).

Die Verbindungen der allgemeinen Struktur (**Ia)** können mittels Palladium katalysierter Reaktionen aus den Reaktionspartnern **4** und **20** hergestellt werden (siehe z.B. WO2009/112845 für U = Borosäureester oder Boronsäure; Angewandte Chemie International Edition, 2013, 52, 615 für U = Zinkhalogenid).

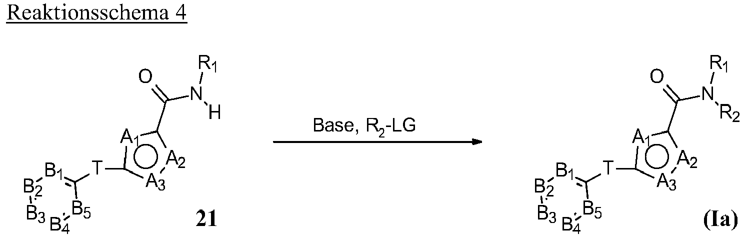

Die Reste A₁, A₂, A₃, B₁, B₂, B₃, B₄, B₅, T und R₁ haben die oben beschriebenen Bedeutungen. R₂ steht für C₁-C₆-Alkyl. LG steht für eien Fluchtgruppe, z.B. Iod oder Brom.

Erfindungsgemäße Verbindungen der allgemeinen Struktur **21** können entsprechend Reaktionsschema 4 durch Deprotonierung mit einer geeigneten Base, wie z.B. Natriumhydrid, und Reaktion mit einem geeigneten Elektrophil, wie z.B. Methyliodid, in erfindungsgemäße Verbindungen der allgemeinen Struktur **(Ia)**, in denen R₂ für C₁-C₆-Alkyl steht, überführt werden.

Das folgende Intermediat (A) zur Herstellung von Verbindungen der Formel **(I)** in denen B₂ und B₄ jeweils für C-H, B₁ und B₅ für C-Cl und B₃ für perfluoriertes Propyl steht, ist bereits aus WO2017/025590 bekannt: 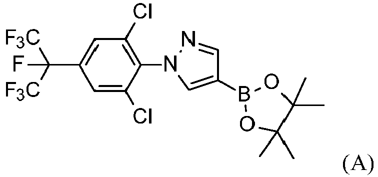 Weiterhin bezieht sich die Erfindung auf die folgenden Intermediate der Formeln (B) und (C) welche zur Herstellung von Verbindungen der Formel **(I),** in denen Z für C, A₁ für CH, A₂ für C-CH₃, A₃ für S, Q für O, R₂ für H und R₁ für Cycloalkyl oder 1-Cyanocyclopropyl steht, eingesetzt werden können.

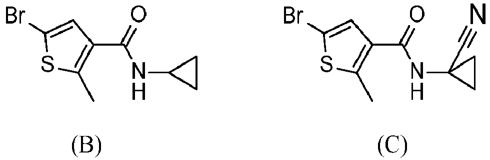

### Experimenteller Teil

### Darstelleung von 1-[2,6-Dichlor-4-(1,1,1,2,3,3,3-heptafluorpropan-2-yl)phenyl]-4-(4,4,5,5-tetramethyl-1,3,2-dioxaborolan-2-yl)-1H-pyrazol

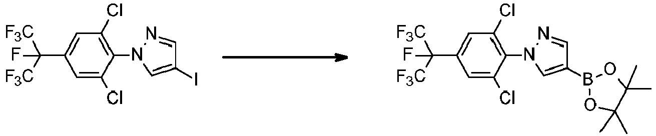

Zu einer Lösung von 1,27 g (2,50 mmol) 1-[2,6-Dichlor-4-(1,1,1,2,3,3,3-heptafluorpropan-2-yl)phenyl]-4-iod-1H-pyrazol in THF wurden bei -39 °C 2,12 mL einer 1,3 M Lösung des Isopropylmagnesiumchlorid Lithiumchlorid-Komplexes getropft und die Reaktionslösung anschließend für 30 min bei -38 °C gerührt. Anschließend wurden 0,59 mL (2,9 mmol) 2-Isopropoxy-4,4,5,5-tetramethyl-1,3,2-dioxaborolan hinzugetropft, das Kühlbad entfernt und die Reaktionslösung für 1 h bei Raumtemperatur gerührt. Es wurde mit gesättigter Ammoniumchlorid-Lösung versetzt und wiederholt mit Cyclohexan extrahiert. Die vereinigten organischen Phasen wurden mit gesättigter Natriumhydrogencarbonat-Lösung und gesättigter Natriumchlorid-Lösung gewaschen, mit Natriumsulfat getrocknet, filtriert und das Lösungsmittel unter reduziertem Druck entfernt. Der Rückstand wurde mittels MPLC an Kieselgel chromatographisch gereinigt (Gradient: Ethylacetat/Cyclohexan 0:100 → 30:70). So wurden 270 mg 1-[2,6-Dichlor-4-(1,1,1,2,3,3,3-heptafluorpropan-2-yl)phenyl]-4-(4,4,5,5-tetramethyl-1,3,2-dioxaborolan-2-yl)-1H-pyrazol erhalten.

¹H-NMR(400.0 MHz, d₆-DMSO):
δ = 8.3610(1.8);8.0447(2.5);7.9563(1.8);3.3155(30.9);2.5234(1.0);2.5099(19.5);2.5057(38.9);2.5012(51. 1);2.4967(37.6);2.4925(18.8);1.3981(3.0);1.2932(16.0);-0.0002(1.8)

### Darstellung von N-(1-Cyancyclopropyl)-2-{1-[2,6-dichlor-4-(1,1,1,2,3,3,3-heptafluorpropan-2-yl)phenyl]-1H-pyrazol-4-yl}-1,3-thiazol-4-carboxamid

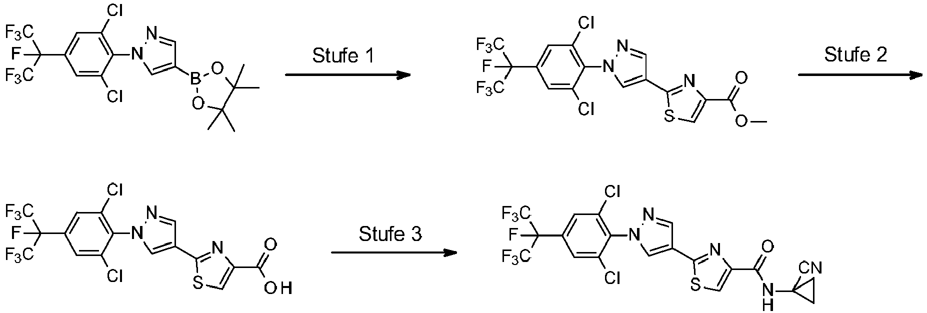

### Stufe 1: Methyl-2-{1-[2,6-dichlor-4-(1,1,1,2,3,3,3-heptafluorpropan-2-yl)phenyl]-1H-pyrazol-4-yl}-1,3-thiazol-4-carboxylat

Eine Mischung von 1,0 g (1,8 mmol) 1-[2,6-Dichlor-4-(1,1,1,2,3,3,3-heptafluorpropan-2-yl)phenyl]-4-(4,4,5,5-tetramethyl-1,3,2-dioxaborolan-2-yl)-1H-pyrazol, 400 mg (1,8 mmol) Methyl-2-brom-1,3-thiazol-4-carboxylat, 416 mg (360 µmol) Tetrakis(triphenylphosphin)palladium, 1,7 g (5,2 mmol) Caesiumcarbonat, 8 mL 1,4-Dioxan und 0,8 mL Wasser wurde für 2 h in einem Mirkowellenreaktor bei 100 °C erhitzt. Das Reaktionsgemisch wurde mit 100 mL Ethylacetat versetzt, mit einer gesättigten wässrigen Natriumchlorid-Lösung gewaschen und anschließend die organische Phase mit Natriumsulfat getrocknet. Es wurde vom Trockenmittel abfiltriert und das Lösungsmittel unter reduziertem Druck entfernt. Der Rückstand wurde mittels präparativer Dünnschichtchromatographie (Gradient: Ethylacetat/Petrolether) chromatographisch aufgetrennt. So wurden 400 mg Methyl-2-{1-[2,6-dichlor-4-(1,1,1,2,3,3,3-heptafluorpropan-2-yl)phenyl]-1H-pyrazol-4-yl}-1,3-thiazol-4-carboxylat erhalten.

¹H-NMR(300.1 MHz, d₆-DMSO):
δ = 8.9335(5.3);8.5257(7.3);8.4429(5.3);8.1134(8.1);6.5446(0.3);3.8618(16.0);3.3211(54.8);2.7275(0.4); 2.5074(54.8);2.5017(71.9);2.4960(51.6);2.2712(0.5);0.0109(0.4);-0.0001(10.4);-0.0111(0.4)

### Stufe 2: 2-{1-[2,6-Dichlor-4-(1,1,1,2,3,3,3-heptafluorpropan-2-yl)phenyl]-1H-pyrazol-4-yl}-1,3-thiazol-4-carbonsäure

Eine Mischung von 420 mg (804 µmol) Methyl-2-{1-[2,6-dichlor-4-(1,1,1,2,3,3,3-heptafluorpropan-2-yl)phenyl]-1H-pyrazol-4-yl}-1,3-thiazol-4-carboxylat und 336 mg (800 µmol) Lithiumhydroxid Monohydrat, 2 mL Wassser und 5 mL Methanol wurde für 4 h bei Raumtemperatur gerührt. Anschließend wurde die Lösung durch die Zugabe von konzentrierter Salzsäure auf einen pH-Wert von 5 - 6 eingestellt und mit Dichlormethan extrahiert. Die vereinigten organischen Phasen wurden mit gesättigter wässriger Natriumchlorid-Lösung gewaschen, mit Natriumsulfat getrocknet und filtriert. Das Lösungsmittel wurde unter reduziertem Druck entfernt. Es wurden 510 mg eines Gemischs der 2-{1-[2,6-Dichlor-4-(1,1,1,2,3,3,3-heptafluorpropan-2-yl)phenyl]-1H-pyrazol-4-yl}-1,3-thiazol-4-carbonsäure mit unbekannten Verunreinigungen erhalten.

¹H-NMR(400.2 MHz, d₆-DMSO):
δ = 8.7673(5.0);8.3477(4.3);8.1073(16.0);7.6718(3.4);4.0228(0.6);4.0064(0.5);3.8629(0.5);3.3328(173.3 );3.3101(5.0);2.8922(0.4);2.7327(0.3);2.6725(1.3);2.5036(214.5);2.3304(1.3);2.0878(0.3);1.2356(2.2);1. 1482(1.3);0.9410(1.4);0.9241(1.4);0.8548(0.4);0.0016(71.0);-0.1479(0.4)

### Stufe 3: N-(1-Cyancyclopropyl)-2-{1-[2,6-dichlor-4-(1,1,1,2,3,3,3-heptafluorpropan-2-yl)phenyl]-1H-pyrazol-4-yl}-1,3-thiazol-4-carboxamid

Eine Lösung von 100 mg des Rückstands aus Stufe 2 in 4 mL N,N-Dimethylformamid wurde mit 35 mg (0,30 mmol) 1-Aminocyclopropancarbonitrilhydrochlorid, 114 mg (300 µmol) N,N,N,N-Tetramethyl-O-(7-azabenzotriazol- 1 -yl)uroniumhexafluorophosphat (HATU) und 52 mg (0,40 mmol) Diispropylethylamin versetzt. Das Reaktionsgemisch wurde für 6 h bei Raumtemperatur gerührt. Anschließend wurde eine gesättigte wässrige Natriumchlorid-Lösung zugesetzt und das Gemisch mit Ethylacetat extrahiert. Die vereinigten organischen Phasen wurden mit gesättigter wässriger Natriumchlorid-Lösung gewaschen, mit Natriumsulfat getrocknet und filtriert. Das Lösungsmittel wurde unter reduziertem Druck entfernt. Der Rückstand wurde mittels HPLC chromatographisch aufgetrennt. So wurden 19 mg N-(1-Cyancyclopropyl)-2- {1-[2,6-dichlor-4-(1,1,1,2,3,3,3-heptafluorpropan-2-yl)phenyl]-1H-pyrazol-4-yl}-1,3-thiazol-4-carboxamid erhalten.

¹H-NMR(300.1 MHz, d₆-DMSO):
δ = 9.3332(5.8);8.9075(0.7);8.8701(10.7);8.4267(10.4);8.3503(14.4);8.1109(16.0);6.5159(3.8);5.7424(1. 3);3.6057(0.6);3.3890(0.8);3.3134(232.3);3.2896(8.2);2.7270(5.2);2.7211(3.8);2.6517(0.7);2.5849(1.2); 2.5065(611.3);2.5007(800.6);2.4951(565.5);2.2766(3.7);2.2704(4.6);2.1633(1.0);2.0899(1.2);2.0495(0.7 );1.5985(2.2);1.5789(5.3);1.5703(5.8);1.5529(2.6);1.3563(3.0);1.3396(5.6);1.3301(5.4);1.3117(2.0);1.23 42(0.9);0.8563(0.6);0.1949(2.2);0.0108(22.0);-0.000 1 (542.6);-0.0 111 (19.3);-0.1987(2.2);-2.2299(0.6);-3.3736(0.6);-3.5153(0.7)

### Herstellung von Methyl-4-{1-[2,6-dichlor-4-(1,1,1,2,3,3,3-heptafluorpropan-2-yl)phenyl]-1H-pyrazol-4-yl}-1-methyl-1H-pyrrol-2-carboxylat

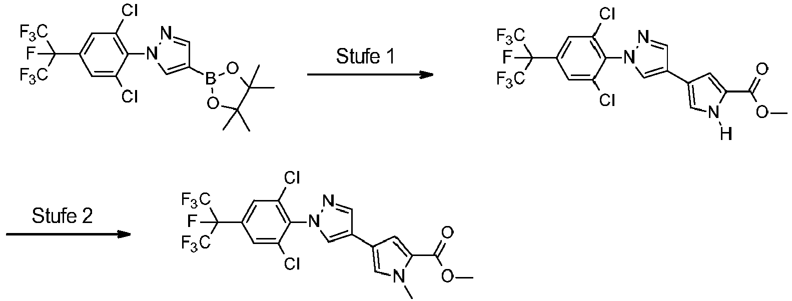

### Stufe 1: Methyl-4-{1-[2,6-dichlor-4-(1,1,1,2,3,3,3-heptafluorpropan-2-yl)phenyl]-1H-pyrazol-4-yl}-1H-pyrrol-2-carboxylat

Eine Mischung von 1,7 g (3,4 mmol) 1-[2,6-Dichlor-4-(1,1,1,2,3,3,3-heptafluorpropan-2-yl)phenyl]-4-(4,4,5,5-tetramethyl-1,3,2-dioxaborolan-2-yl)-1H-pyrazol, 3,0 g (9,9 mmol) 1-tert-Butyl-2-methyl-4-brom-1H-pyrrol-1,2-dicarboxylat, 763 mg (660 µmol) Tetrakis(triphenylphosphin)palladium, 3,2 g (9,8 mmol) Caesiumcarbonat, 20 mL 1,4-Dioxan und 2 mL Wasser wurde für 2 h in einem Mirkowellenreaktor bei 100 °C erhitzt. Das Reaktionsgemisch wurde mit 100 mL Ethylacetat versetzt, mit einer gesättigten wässrigen Natriumchlorid-Lösung gewaschen und anschließend die organische Phase mit Natriumsulfat getrocknet. Es wurde vom Trockenmittel abfiltriert und das Lösungsmittel unter reduziertem Druck entfernt. Der Rückstand wurde mittels präparativer Dünnschichtchromatographie (Gradient: Ethylacetat/Petrolether) chromatographisch aufgetrennt. So wurden 600 mg Methyl-4-{1-[2,6-dichlor-4-(1,1,1,2,3,3,3-heptafluorpropan-2-yl)phenyl]-1H-pyrazol-4-yl}-1H-pyrrol-2-carboxylat.

¹H-NMR(300.1 MHz, d₆-DMSO):
δ = 11.9847(1.1);8.3084(5.9);8.1316(5.9);8.0531(8.7);7.3581(1.8);7.3527(2.2);7.3489(2.2);7.3435(1.8);7 .0571(2.0);7.0512(2.8);7.0442(1.8);6.5190(0.4);3.7806(16.0);3.3136(68.4);2.7268(0.5);2.5066(60.6);2.5 010(77.6);2.4954(55.0);2.2764(0.4);2.2711(0.5);1.2355(1.5);1.0688(0.8);0.0105(0.5);-0.0001(10.3)

### Stufe 2: Methyl-4-{1-[2,6-dichlor-4-(1,1,1,2,3,3,3-heptafluorpropan-2-yl)phenyl]-1H-pyrazol-4-yl}-1-methyl-1H-pyrrol-2-carboxylat

Eine Lösung von 600 mg (1,19 mmol) (Methyl-4-{1-[2,6-dichlor-4-(1,1,1,2,3,3,3-heptafluorpropan-2-yl)phenyl]-1H-pyrazol-4-yl}-1H-pyrrol-2-carboxylatin 5 mL N,N-Dimethylformamid wurde bei 0 °C mit 58 mg (2,4 mmol) Natriumhydrid versetzt und 15 min bei dieser Temperatur gerührt. Anschließend wurden 255 mg (1,80 mmol) Methyliodid zugesetzt. Das Reaktionsgemisch wurde für 6 h bei Raumtemperatur gerührt, mit Eiswasser versetzt und mehrmals mit Ethylacetat extrahiert. Die vereinigten organischen Phasen wurden mit einer gesättigten wässrigen Natriumchloridlösung gewaschen, mit Natriumsulfat getrocknet, filtriert und das Lösungsmittel unter reduziertem Druck entfernt. Der Rückstand wurde mittels präparativer Dünnschichtchromatographie (Gradient: Ethylacetat/Petrolether) chromatographisch aufgetrennt. So wurden 400 mg Methyl-4-{1-[2,6-dichlor-4-(1,1,1,2,3,3,3-heptafluorpropan-2-yl)phenyl]-1H-pyrazol-4-yl}-1-methyl-1H-pyrrol-2-carboxylat erhalten.

¹H-NMR(300.1 MHz, d₆-DMSO):
δ = 8.2975(5.4);8.0894(5.4);8.0585(8.0);7.4203(2.8);7.4140(2.9);7.1076(3.6);7.1010(3.5);4.1281(0.4);4. 1107(1.1);4.0932(1.1);4.0759(0.4);3.8832(14.4);3.7625(16.0);3.7393(0.4);3.3300(7.6);3.1773(4.9);3.15 99(5.3);2.5143(7.4);2.5086(14.6);2.5028(19.3);2.4970(13.6);-0.0001(4.1)

### Herstellung von Ethyl-2-brom-5-{1-[2,6-dichlor-4-(1,1,1,2,3,3,3-heptafluorpropan-2-yl)phenyl]-1H-pyrazol-4-yl}thiophen-3-carboxylat

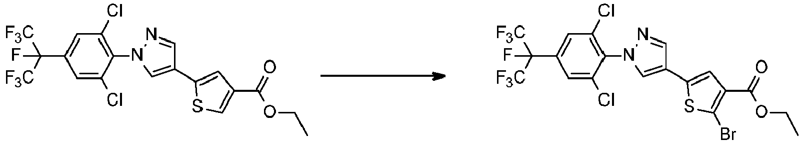

Eine Lösung von 500 mg (959 µmol) Ethyl-5-{1-[2,6-dichlor-4-(1,1,1,2,3,3,3-heptafluorpropan-2-yl)phenyl]-1H-pyrazol-4-yl}thiophen-3-carboxylat in 3 mL Essigsäure wurde mit 130 mg (730 µmol) N-Bromsuccinimid versetzt. Das Reaktionsgemisch wurde für 30 min bei Raumtemperatur gerührt, mit einer gesättigten wässrigen Kochsalzlösung versetzt und mehrmals mit Ethylacetat extrahiert. Die vereinigten organischen Phasen wurden mit einer gesättigten wässrigen Natriumchloridlösung gewaschen, mit Natriumsulfat getrocknet, filtriert und das Lösungsmittel unter reduziertem Druck entfernt. Der Rückstand wurde mittels Säulenchromatographie an Kieselgel (Gradient: Ethylacetat/Petrolether) chromatographisch aufgetrennt. So wurden 390 mg Ethyl-2-brom-5-{1-[2,6-dichlor-4-(1,1,1,2,3,3,3-heptafluorpropan-2-yl)phenyl]-1H-pyrazol-4-yl}thiophen-3-carboxylat erhalten.

¹H-NMR(300.1 MHz, d₆-DMSO):
δ = 8.6631(8.8);8.6452(0.5);8.3343(8.9);8.0962(13.3);7.5786(11.6);6.5358(1.0);4.3404(2.1);4.3168(6.8); 4.2932(7.0);4.2696(2.2);3.3266(121.6);2.7345(0.6);2.7279(0.7);2.5079(93.7);2.5021(122.5);2.4964(85.7 );2.2708(0.7);1.3710(0.4);1.3510(7.6);1.3274(16.0);1.3037(7.2);1.2356(0.6);0.0109(0.8);-0.0001(20.1)

### Herstellung von 5-Chlor-N-cyclopropyl-4-{1-[2,6-dichlor-4-(1,1,1,2,3,3,3-heptafluorpropan-2-yl)phenyl]-1H-pyrazol-4-yl}-1-methyl-1H-pyrrol-2-carboxamid

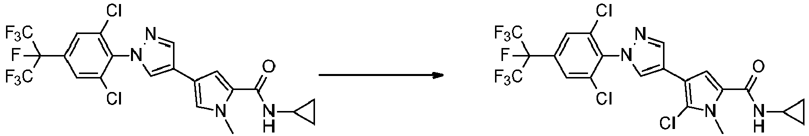

Eine Lösung von 45 mg (83 µmol) N-Cyclopropyl-4-{1-[2,6-dichlor-4-(1,1,1,2,3,3,3-heptafluorpropan-2-yl)phenyl]-1H-pyrazol-4-yl}-1-methyl-1H-pyrrol-2-carboxamid in 0,3 mL Essigsäure wurden bei 0 °C mit 10 mg (75 µmol) N-Chlorsuccinimid versetzt. Das Reaktionsgemisch wurde für 30 min bei Raumtemperatur gerührt, mit einer gesättigten wässrigen Natriumthiosulfatlösung versetzt und erneut 30 min bei Raumtemperatur gerührt. Anschließend wurde mit gesättigter wässriger Kochsalzlösung versetzt und mehrmals mit Ethylacetat extrahiert. Die vereinigten organischen Phasen wurden mit einer gesättigten wässrigen Natriumchloridlösung gewaschen, mit Natriumsulfat getrocknet, filtriert und das Lösungsmittel unter reduziertem Druck entfernt. Der Rückstand wurde mittels HPLC chromatographisch aufgetrennt. So wurden 13 mg 5-Chlor-N-cyclopropyl-4-{1-[2,6-dichlor-4-(1,1,1,2,3,3,3-heptafluorpropan-2-yl)phenyl]-1H-pyrazol-4-yl}-1-methyl-1H-pyrrol-2-carboxamid erhalten.

¹H-NMR(400.0 MHz, d₆-DMSO):
δ = 8.3976(5.6);8.1968(1.5);8.1869(1.5);8.0746(5.5);8.0489(8.4);7.0532(5.8);3.8637(16.0);3.3216(75.3); 2.9610(0.3);2.7964(0.5);2.7869(0.8);2.7783(1.1);2.7685(1.2);2.7600(0.8);2.7505(0.5);2.6749(0.5);2.670 6(0.7);2.6663(0.5);2.5061(88.6);2.5017(116.5);2.4973(85.5);2.3284(0.7);2.3237(0.5);2.0742(6.1);0.700 6(0.7);0.6877(1.9);0.6826(2.7);0.6706(2.5);0.6645(2.2);0.6534(0.9);0.5535(0.9);0.5430(2.8);0.5369(2.5) ;0.5275(2.2);0.5152(0.7);0.0079(0.7);-0.0002(18.7)

### Herstellung von 2-Chlor-N-cyclopropyl-5-{1-[2,6-dichlor-4-(1,1,1,2,3,3,3-heptafluorpropan-2-yl)phenyl]-1H-pyrazol-4-yl}thiophen-3-carboxamid

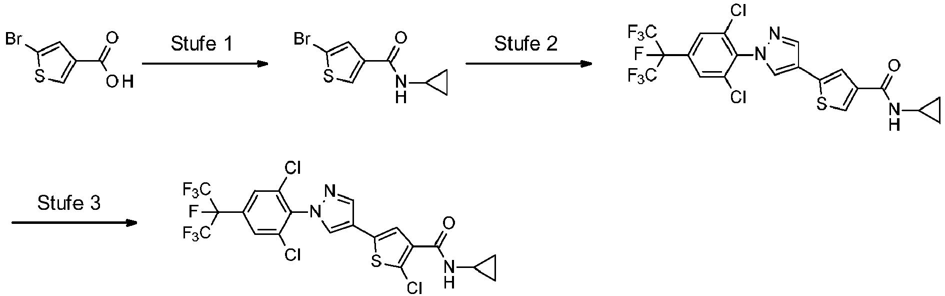

### Stufe 1: 5-Brom-N-cyclopropylthiophen-3-carboxamid

Eine Lösung von 1,00 g (4,83 mmol) 5-Bromthiophen-3-carbonsäure in 20 mL CH₂Cl₂ wurde mit einem Tropfen N,N-Dimethylformamid und 1,0 mL (11 mmol) Oxalsäuredichlorid versetzt. Die Lösung wurde für 3 h unter Rückfluss erhitzt, daraufhin erneut 1,0 mL (11 mmol) Oxalsäuredichlorid hinzugefügt und weitere 1,5 h unter Rückfluss erhitzt. Anschließend wurde das Lösungsmittel unter reduziertem Druck entfernt.

Der Rückstand wurde in 20 mL CH₂Cl₂ gelöst, bei 0 °C mit 0,75 mL (5,4 mmol) Triethylamin und 0,37 mL (5,3 mmol) Cyclopropylamin versetzt und anschließend über Nacht bei Raumtemperatur gerührt. Die Reaktionslösung wurde mit CH₂Cl₂ verdünnt und mehrmals mit Wasser gewaschen. Die organische Phase wurde mit Natriumsulfat getrocknet, filtriert und das Lösungsmittel unter reduziertem Druck entfernt. Der Rückstand wurde mittels MPLC an RP-Kieselgel (Gradient: H₂O/Acetonitril) chromatographisch aufgetrennt. So wurden 1,06 g 5-Brom-N-cyclopropylthiophen-3-carboxamid erhalten.

¹H-NMR(400.0 MHz, d₆-DMSO):
δ = 8.2864 (4.0); 8.2781 (4.0); 8.0736 (15.3); 8.0696 (15.5); 7.5558 (16.0); 7.5517 (15.8); 3.3238 (22.1); 2.8055 (0.8); 2.7956 (2.4); 2.7861 (3.3); 2.7774 (5.4); 2.7676 (5.4); 2.7590 (3.4); 2.7495 (2.5); 2.7395 (0.9); 2.6718 (0.4); 2.5250 (1.0); 2.5116 (27.4); 2.5072 (55.9); 2.5028 (73.5); 2.4983 (52.4); 2.4940 (25.1); 2.3339 (0.3); 2.3294 (0.4); 2.0756 (0.7); 0.7038 (3.0); 0.6915 (8.8); 0.6859 (11.8); 0.6739 (11.6); 0.6679 (8.9); 0.6566 (4.3); 0.6354 (0.6); 0.6174 (0.6); 0.5848 (0.6); 0.5747 (0.6); 0.5452 (4.2); 0.5347 (11.7); 0.5280 (10.3); 0.5245 (9.5); 0.5185 (9.2); 0.5065 (2.9); -0.0002 (3.2)

### Stufe 2: N-Cyclopropyl-5-{1-[2,6-dichlor-4-(1,1,1,2,3,3,3-heptafluorpropan-2-yl)phenyl]-1H-pyrazol-4-yl}thiophen-3-carboxamid

Eine Mischung von 380 mg (749 µmol) 1-[2,6-Dichlor-4-(1,1,1,2,3,3,3-heptafluorpropan-2-yl)phenyl]-4-(4,4,5,5-tetramethyl-1,3,2-dioxaborolan-2-yl)-1H-pyrazol, 185 mg (752 µmol) 5-Brom-N-cyclopropylthiophen-3-carboxamid, 173 mg (150 µmol) Tetrakis(triphenylphosphin)palladium, 735 mg (2,25 mmol) Caesiumcarbonat, 4 mL 1,4-Dioxan und 0,4 mL Wasser wurde für 2 h in einem Mirkowellenreaktor bei 100 °C erhitzt. Anschließend wurde das Lösungsmittel im Vakuum entfernt. Der Rückstand wurde mit Wasser versetzt und mit Ethylacetat extrahiert. Die vereinigten organischen Phasen wurden mit Natriumsulfat getrocknet, filtriert und das Lösungsmittel unter reduziertem Druck entfernt. Der Rückstand wurde mittels MPLC an Kieselgel (Gradient: Ethylacetat/Cyclohexan) chromatographisch aufgetrennt. So wurden 262 mg N-Cyclopropyl-5-{1-[2,6-dichlor-4-(1,1,1,2,3,3,3-heptafluorpropan-2-yl)phenyl]-1H-pyrazol-4-yl}thiophen-3-carboxamid erhalten.

¹H-NMR(400.0 MHz, d₆-DMSO):
δ = 8.5643 (10.2); 8.3084 (3.2); 8.2986 (3.3); 8.2151 (10.4); 8.0821 (16.0); 7.9803 (7.3); 7.6416 (7.2); 3.9294 (0.8); 3.3239 (34.4); 2.8354 (0.3); 2.8251 (1.0); 2.8159 (1.5); 2.8070 (2.2); 2.7975 (2.2); 2.7888 (1.5); 2.7794 (1.0); 2.7690 (0.4); 2.6719 (0.5); 2.5066 (70.2); 2.5027 (90.6); 2.4990 (68.6); 2.3299 (0.5); 1.0695 (4.9); 0.7210 (1.2); 0.7080 (3.8); 0.7032 (5.2); 0.6910 (4.8); 0.6853 (4.2); 0.6742 (1.7); 0.5698 (1.7); 0.5593 (5.3); 0.5528 (5.1); 0.5438 (4.4); 0.5313 (1.2); 0.0000 (9.6)

### Stufe 3: 2-Chlor-N-cyclopropyl-5-{1-[2,6-dichlor-4-(1,1,1,2,3,3,3-heptafluorpropan-2-yl)phenyl]-1H-pyrazol-4-yl}thiophen-3-carboxamid

Eine Lösung von 50 mg (86 µmol) N-Cyclopropyl-5-{1-[2,6-dichlor-4-(1,1,1,2,3,3,3-heptafluorpropan-2-yl)phenyl]-1H-pyrazol-4-yl}thiophen-3-carboxamid in 2 mL Essigsäure wurde bei 10 °C innerhalb von 30 min mit einer Lösung von 13 mg (97 µmol) N-Chlorsuccinimid in 1 mL Essigsäure versetzt. Das Reaktionsgemisch wurde für 18 h bei 60 °C gerührt, mit einer gesättigten wässrigen Natriumthiosulfatlösung versetzt und erneut 30 min bei Raumtemperatur gerührt. Anschließend wurde mit gesättigter wässriger Kochsalzlösung versetzt und mehrmals mit Ethylacetat extrahiert. Die vereinigten organischen Phasen wurden mit Natriumsulfat getrocknet, filtriert und das Lösungsmittel unter reduziertem Druck entfernt. Der Rückstand wurde mittels HPLC (Gradient: H₂O/Acetonitril) chromatographisch aufgetrennt. So wurden 31 mg 2-Chlor-N-cyclopropyl-5-{1-[2,6-dichlor-4-(1,1,1,2,3,3,3-heptafluorpropan-2-yl)phenyl]-1H-pyrazol-4-yl}thiophen-3-carboxamid erhalten.

¹H-NMR(400.0 MHz, d₆-DMSO):
δ = 9.1968 (0.4); 8.7769 (1.0); 8.7466 (0.4); 8.5761 (9.3); 8.5621 (0.6); 8.3702 (2.5); 8.3595 (2.6); 8.2724 (1.1); 8.2292 (9.3); 8.2007 (0.6); 8.0858 (16.0); 7.8646 (0.6); 7.4156 (10.1); 7.3687 (0.6); 5.7567 (12.1); 3.3217 (86.6); 2.8437 (0.4); 2.8330 (1.0); 2.8241 (1.4); 2.8148 (2.0); 2.8052 (2.0); 2.7964 (1.3); 2.7863 (0.9); 2.7770 (0.4); 2.6712 (0.9); 2.5062 (129.2); 2.5020 (170.7); 2.4979 (125.2); 2.3284 (0.9); 2.0744 (2.6); 1.5860 (0.4); 1.5797 (0.4); 1.2624 (0.4); 1.2569 (0.4); 0.7379 (0.5); 0.7270 (1.4); 0.7146 (3.3); 0.7093 (4.5); 0.6971 (4.3); 0.6913 (3.6); 0.6802 (1.5); 0.5629 (1.5); 0.5522 (4.5); 0.5455 (4.3); 0.5363 (3.9); 0.5240 (1.4); 0.5069 (0.5); 0.1463 (0.3); 0.0077 (2.5); -0.0002 (68.7); -0.1495 (0.3)

### Herstellung von 2-Chlor-N-(1-cyancyclopropyl)-5-{1-[2,6-dichlor-4-(1,1,1,2,3,3,3-heptafluorpropan-2-yl)phenyl]-1H-pyrazol-4-yl}-N-methylthiophen-3-carboxamid

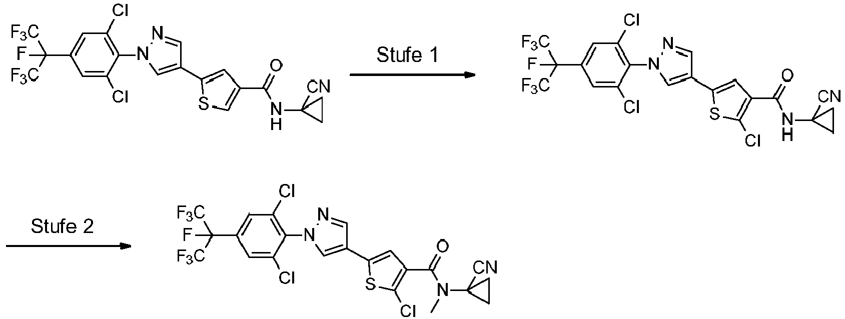

### Stufe 1: 2-Chlor-N-(1-cyancyclopropyl)-5-{1-[2,6-dichlor-4-(1,1,1,2,3,3,3-heptafluorpropan-2-yl)phenyl]-1H-pyrazol-4-thiophen-3-carboxamid

Eine Lösung von 70 mg (0,12 mmol) N-(1-Cyancyclopropyl)-5-{1-[2,6-dichlor-4-(1,1,1,2,3,3,3-heptafluorpropan-2-yl)phenyl]-1H-pyrazol-4-yl}thiophen-3-carboxamid in 1 mL Chloroform wurde mit 32 mg (0,14 mmol) 2-Chloro-1,3-bis(methoxycarbonyl)guanidin versetzt und über Nacht bei Raumtemperatur gerührt. Das Lösungsmittel wurde anschließend unter reduziertem Druck entfernt und der Rückstand mittels HPLC (Gradient: H₂O/Acetonitril) chromatographisch aufgetrennt. So wurden 60 mg 2-Chlor-N-(1-cyancyclopropyl)-5-{1-[2,6-dichlor-4-(1,1,1,2,3,3,3-heptafluorpropan-2-yl)phenyl]-1H-pyrazol-4-yl}thiophen-3-carboxamid erhalten.

¹H-NMR(400.0 MHz, d₆-DMSO):
δ = 9.6859 (1.5); 9.2607 (5.2); 8.8019 (2.3); 8.5964 (8.8); 8.2949 (2.4); 8.2366 (8.9); 8.0890 (16.0); 7.4870 (10.3); 3.3210 (33.2); 2.6752 (0.5); 2.6709 (0.7); 2.6665 (0.5); 2.5240 (1.7); 2.5104 (42.9); 2.5062 (89.1); 2.5018 (119.6); 2.4974 (86.3); 2.4932 (41.6); 2.3289 (0.6); 2.3237 (0.5); 1.6520 (0.5); 1.6379 (1.3); 1.6310 (1.3); 1.6175 (0.6); 1.5987 (1.9); 1.5843 (4.8); 1.5774 (5.1); 1.5644 (2.2); 1.2817 (2.2); 1.2682 (4.8); 1.2615 (5.2); 1.2469 (2.4); 1.2329 (1.4); 1.2262 (1.4); 1.2118 (0.5); 0.9264 (0.3); 0.9097 (0.3); 0.0077 (1.6); -0.0004 (42.8); -0.0086 (1.6)

### Stufe 2: 2-Chlor-N-(1-cyancyclopropyl)-5-{1-[2,6-dichlor-4-(1,1,1,2,3,3,3-heptafluorpropan-2-yl)phenyl]-1H-pyrazol-4-yl}-N-methylthiophen-3-carboxamid

Zu einer Lösung von 50 mg (83 µmol) 2-Chlor-N-(1-cyancyclopropyl)-5-{1-[2,6-dichlor-4-(1,1,1,2,3,3,3-heptafluorpropan-2-yl)phenyl]-1H-pyrazol-4-yl}thiophen-3-carboxamid in Tetrahydrofuran wurde bei 0 °C 5 mg (0,1 mmol) Natriumhydrid (55%ige Dispersion in Mineralöl) gegeben. Die Suspension wurde für 10 min bei 0 °C gerührt, anschließend mit 7 µL (0,1 mmol) Methyliodid versetzt und über Nacht bei Raumtemperatur gerührt. Die Reaktionslösung wurde mit CH₂Cl₂ verdünnt und mit Wasser gewaschen. Die organischen Phase wurde mit Natriumsulfat getrocknet, filtriert und das Lösungsmittel unter reduziertem Druck entfernt. Der Rückstand wurde mittels HPLC (Gradient: H₂O/Acetonitril) chromatographisch aufgetrennt. So wurden 25 mg 2-Chlor-N-(1-cyancyclopropyl)-5-{1-[2,6-dichlor-4-(1,1,1,2,3,3,3-heptafluorpropan-2-yl)phenyl]-1H-pyrazol-4-yl}-N-methylthiophen-3-carboxamid erhalten.

¹H-NMR(400.0 MHz, d₆-DMSO):
δ = 8.8156 (1.0); 8.6100 (9.3); 8.3161 (1.2); 8.2652 (8.4); 8.0870 (16.0); 7.3933 (12.2); 5.7553 (1.7); 3.3194 (128.0); 3.0327 (13.4); 2.9944 (2.9); 2.6749 (1.2); 2.6706 (1.8); 2.6665 (1.3); 2.5240 (4.5); 2.5104 (109.0); 2.5061 (227.3); 2.5017 (304.5); 2.4973 (220.5); 2.4934 (108.7); 2.3327 (1.3); 2.3285 (1.7); 2.3244 (1.3); 2.0739 (0.4); 2.0084 (0.4); 1.9891 (0.4); 1.7318 (0.4); 1.7190 (0.5); 1.7076 (0.4); 1.6376 (2.3); 1.4846 (0.4); 1.4441 (2.3); 1.4305 (4.3); 1.4245 (4.4); 1.4085 (1.4); 1.2354 (3.1); 0.8542 (0.7); 0.1463 (0.8); 0.0079 (5.5); -0.0001 (165.6); -0.0084 (7.0); -0.1495 (0.8)

**Tabelle I-T1**

| 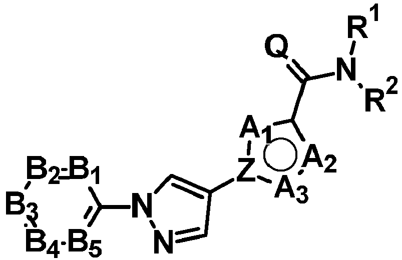 B₂ und B₄ = CH | | | | | | | | | | |
|---|---|---|---|---|---|---|---|---|---|---|
| **Bsp.-Nr.** | **B₁** | **B₃** | **B₅** | **R₁** | **R₂** | **Z** | **A₁** | **A₂** | **A₃** | **Q** |
| I-T1-1 | C-Cl | C-i-C₃F₇ | C-Cl | cyclopropyl | H | C | C-H | C-Br | s | O |
| I-T1-2 | C-Cl | C-i-C₃F₇ | C-Cl | 1 -(cyano)cyclopropyl | H | C | C-H | C-Br | s | O |
| I-T1-3 | C-Cl | C-i-C₃F₇ | C-Cl | cyclopropyl | H | C | C-H | N-CH₃ | C-H | O |
| I-T1-4 | C-Cl | C-i-C₃F₇ | C-Cl | 1 -(cyano)cyclopropyl | H | C | C-H | N-CH₃ | C-H | O |
| I-T1-5 | C-Cl | C-i-C₃F₇ | C-Cl | 1 -(cyano)cyclopropyl | H | C | N | C-H | s | O |
| I-T1-6 | C-Cl | C-i-C₃F₇ | C-Cl | benzyl | H | C | C-H | C-Br | s | O |

| **Bsp.-Nr.** | **B₁** | **B₃** | **B₅** | **R₁** | **R₂** | **Z** | **A₁** | **A₂** | **A₃** | **Q** |
|---|---|---|---|---|---|---|---|---|---|---|
| I-T1-7 | C-Cl | C-i-C₃F₇ | C-Cl | cyclopropyl | H | C | N | C-Cl | s | O |
| I-T1-8 | C-Cl | C-i-C₃F₇ | C-Cl | cyclopropyl | H | C | N | C-H | s | O |
| I-T1-9 | C-Cl | C-i-C₃F₇ | C-Cl | cyclopropyl | H | C | C-H | N-CH₃ | C-Cl | O |
| I-T1-10 | C-Cl | C-i-C₃F₇ | C-Cl | 1 -(cyano)cyclopropyl | H | C | C-H | C-CH₃ | s | O |
| I-T1-11 | C-Cl | C-i-C₃F₇ | C-Cl | cyclopropyl | H | C | C-H | C-CH₃ | s | O |
| I-T1-12 | C-Cl | C-i-C₃F₇ | C-Cl | cyclopropyl | H | C | C-H | C-H | s | O |
| I-T1-13 | C-Cl | C-i-C₃F₇ | C-Cl | 1 -(cyano)cyclopropyl | H | C | C-H | C-H | s | O |
| I-T1-14 | C-Cl | C-i-C₃F₇ | C-Cl | cyclopropyl | H | C | C-H | C-I | s | O |
| I-T1-15 | C-Cl | C-i-C₃F₇ | C-Cl | 1 -(cyano)cyclopropyl | H | C | C-H | C-I | s | O |
| I-T1-16 | C-Cl | C-i-C₃F₇ | C-Cl | cyclopropyl | H | C | C-H | C-Cl | s | O |
| I-T1-17 | C-Cl | C-i-C₃F₇ | C-Cl | 1 -(cyano)cyclopropyl | H | C | C-H | C-Cl | s | O |
| I-T1-18 | C-Cl | C-i-C₃F₇ | C-Cl | 1 -(cyano)cyclopropyl | CH₃ | C | C-H | C-Cl | s | O |

### NMR-Daten ausgewählter Beispiele

### NMR-Peak-Listenverfahren

Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ ₁ (Intensität₁⁾; δ₂ (Intensität₂); ..; δᵢ (Intensitätᵢ⁾; ; δₙ (Intensität.)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel I-T1-1: ¹H-NMR (400 MHz, d₆-DMSO): |
| δ= 8.5815(8.5);8.4144(0.5);8.3785(4.3);8.3694(4.1);8.2844(0.5);8.2324(8.6);8.0957(16.0);7.3923(9.1);3.4571 (0.3);3.3327(46 .0);2.9359(0.3);2.8979(0.4);2.8146(3.1);2.8051(3.1);2.6762(2.7);2.5053(283.4);2.3309(2.4);1.9794(0.5);1.9616(0.4);1.4446(0 .4);1.2380(5.2);1.1519(0.4);0.8558(0.9);0.8393(0.5);0.8108(0.4);0.7124(6.7);0.6989(6.6);0.6431(0.6);0.5478(7.8);0.3374(0.3) ;0.1485(0.7);0.0030(75.4);-0.1475(0.6) |
| Beispiel I-T1-2: ¹H-NMR(300.1 MHz, d₆-DMSO): |
| δ=9.2672(6.6);8.5953(10.3);8.2352(10.3);8.0937(16.0);7.7245(0.4);7.6933(0.4);7.4616(12.2);4.0248(0.9);4.0027(0.9);3.3259 (50.1);2.7279(0.5);2.5076(67.5);2.5019(87.0);2.4962(61.3);2.2713(0.6);2.0757(7.8);1.6085(2.3);1.5894(5.7);1.5801(5.9);1.56 29(2.6);1.3333(0.4);1.2820(2.9);1.2642(5.9);1.2551(6.0);1.2357(3.1);1.1385(0.4);1.1153(0.5);0.9425(2.6);0.9200(2.6);0.0107 (2.6);-0.0001(58.8);-0.0111(2.2) |
| Beispiel I-T1-3: ¹H-NMR(400.2 MHz, d₆-DMSO): |
| δ= 8.1742(5.8);8.0629(1.9);8.0467(9.7);7.9512(5.8);7.1863(3.3);7.1821(3.4);6.9048(3.4);6.9003(3.4);6.5313(0.7);3.8492(16. 0);3.3241(43.4);3.3005(1.9);2.7787(0.6);2.7689(0.8);2.7606(1.3);2.7509(1.2);2.7425(0.8);2.7329(0.6);2.6711(0.5);2.5066(60. 9);2.5023(80.1);2.4980(59.3);2.3291(0.6);2.0759(0.9);1.1475(0.5);0.6780(0.9);0.6647(2.2);0.6599(3.0);0.6481(2.7);0.6421(2. 3);0.6312(1.0);0.5388(1.1);0.5283(3.2);0.5218(2.9);0.5130(2.4);0.5007(0.7);0.0007(30.5) |
| Beispiel I-T1-4: ¹H-NMR(300.1 MHz, d₆-DMSO): |
| δ= 8.9155(3.6);8.3831(0.4);8.2068(6.3);8.0499(9.4);7.9672(6.4);7.2960(3.3);7.2906(3.4);6.9945(3.4);6.9884(3.4);6.5453(1.4) ;3.8791(16.0);3.3211(95.6);3.2977(0.8);2.7273(1.2);2.7210(0.9);2.5128(77.7);2.5071(151.3);2.5013(199.3);2.4955(139.3);2.2 772(0.9);2.2715(1.2);2.2653(0.9);1.5457(1.2);1.5267(3.2);1.5175(3.4);1.5007(1.6);1.2927(1.4);1.2515(1.6);1.2343(3.6);1.225 1(3.4);1.2061(1.2);1.0679(0.7);0.0108(1.1);-0.0001(32.6);-0.0112(1.1) |
| Beispiel I-T1-5: ¹H-NMR(300.1 MHz, d₆-DMSO): |
| δ= 9.3332(5.8);8.9075(0.7);8.8701(10.7);8.4267(10.4);8.3503(14.4);8.1109(16.0);6.5159(3.8);5.7424(1.3);3.6057(0.6);3.3890 (0.8);3.3134(232.3);3.2896(8.2);2.7270(5.2);2.7211(3.8);2.6517(0.7);2.5849(1.2);2.5065(611.3);2.5007(800.6);2.4951(565.5) ;2.2766(3.7);2.2704(4.6);2.1633(1.0);2.0899(1.2);2.0495(0.7);1.5985(2.2);1.5789(5.3);1.5703(5.8);1.5529(2.6);1.3563(3.0);1. 3396(5.6); 1.330 1(5.4); 1.3117(2.0); 1.2342(0. 9);0.8563(0.6);0.1949(2.2);0.0 108(22.0);-0.0001 (542.6);-0.0111 (19.3);-0.1987(2.2);-2.2299(0.6);-3.3736(0.6);-3.5153(0.7) |
| Beispiel I-T1-6: ¹H-NMR(300.1 MHz, d₆-DMSO): |
| δ= 8.9117(1.5);8.8923(2.8);8.8726(1.4);8.5969(9.0);8.2411(9.0);8.0908(14.1);7.4878(9.8);7.3604(16.0);7.3486(11.2);7.3447( 11.5);7.3243(1.0);7.3159(1.2);7.3003(0.5);7.2852(1.5);7.2743(1.8);7.2644(1.7);7.2565(2.0);7.2479(1.2);4.4546(6.5);4.4348(6 .3);3.3321(56.3);2.5076(33.8);2.5020(42.7);2.4965(30.7);2.0754(0.5);1.2332(0.5);-0.0002(25.8) |
| Beispiel I-T1-7: ¹H-NMR(300.1 MHz, d₆-DMSO): |
| δ= 12.6233(0.5);8.8976(11.3);8.8958(11.3);8.4627(11.3);8.4609(11.2);8.4192(2.9);8.4055(3.2);8.1165(16.0);6.5302(5.0);3.41 87(1.6);3.3239(762.4);3.3007(7.4);3.2233(0.8);2.8662(1.2);2.8516(1.3);2.8426(2.2);2.8285(1.9);2.8047(1.2);2.7272(2.3);2.72 13(1.8);2.5900(1.1);2.5133(143.4);2.5074(283.9);2.5014(375.6);2.4955(256.2);2.4897(115.9);2.3865(0.6);2.2774(1.8);2.2712 (2.3);2.0859(1.0);1.2400(0.9);1.1464(2.2);0.7543(1.3);0.7300(4.7);0.7041(3.7);0.6911(2.5);0.6661(0.8);0.6414(2.4);0.6268(5. 5);0.6140(4.6);0.5894(1.1);0.1952(0.7);0.0108(7.5);-0.0001(193.4);-0.0111(5.8);-0.1980(0.9) |
| Beispiel I-T1-8: ¹H-NMR(300.1 MHz, d₆-DMSO): |
| δ= 8.8655(10.9);8.4340(10.8);8.2977(2.9);8.2832(2.9);8.1991(14.8);8.1077(16.0);6.5160(0.5);3.3135(35.2);3.2897(1.2);2.908 1(0.4);2.8950(0.9);2.8824(1.3);2.8703(1.9);2.8573(1.8);2.8331(0.9);2.8188(0.3);2.7270(0.7);2.5123(49.1);2.5068(95.0);2.501 0(125.0);2.4952(87.5);2.2706(0.8);2.0737(0.6);1.2348(0.4);0.7523(1.0);0.7273(4.2);0.7080(3.8);0.7017(3.6);0.6896(3.0);0.66 44(2.4);0.6510(6.5);0.6410(3.8);0.6126(1.1);0.1952(0.4);0.0108(4.0);0.0000(99.0);-0.0111 (3.5);-0.1990(0.4) |
| Beispiel I-T1-9: ¹H-NMR(400.0 MHz, d₆-DMSO): |
| δ= 8.3976(5.6);8.1968(1.5);8.1869(1.5);8.0746(5.5);8.0489(8.4);7.0532(5.8);3.8637(16.0);3.3216(75.3);2.9610(0.3);2.7964(0 .5);2.7869(0.8);2.7783(1.1);2.7685(1.2);2.7600(0.8);2.7505(0.5);2.6749(0.5);2.6706(0.7);2.6663(0.5);2.5061(88.6);2.5017(11 6.5);2.4973(85.5);2.3284(0.7);2.3237(0.5);2.0742(6.1);0.7006(0.7);0.6877(1.9);0.6826(2.7);0.6706(2.5);0.6645(2.2);0.6534(0 .9);0.5535(0.9);0.5430(2.8);0.5369(2.5);0.5275(2.2);0.5152(0.7);0.0079(0.7);-0.0002(18.7) |
| Beispiel I-T1-10: ¹H-NMR(400.0 MHz, d₆-DMSO): |
| δ= 9.0102 (3.5); 8.4825 (5.9); 8.1252 (5.9); 8.0772 (8.9); 7.4589 (5.9); 3.3226 (23.0); 2.6712 (0.4); 2.6668 (0.3); 2.6337 (16.0); 2.5242 (0.9); 2.5065 (42.6); 2.5021 (56.7); 2.4977 (41.8); 2.0862 (0.9); 2.0749 (4.8); 1.5678 (1.3); 1.5536 (3.3); 1.5466 (3.6); 1.5338 (1.5); 1.2738 (1.5); 1.2603 (3.4); 1.2536 (3.6); 1.2391 (1.3); 0.0077 (2.3); -0.0002 (62.2); -0.0083 (2.6) |
| Beispiel I-T1-11: ¹H-NMR(400.0 MHz, d₆-DMSO): |
| δ= 8.4543 (5.8); 8.1228 (1.8); 8.1142 (7.3); 8.0723 (8.9); 7.4114 (6.0); 3.3220 (28.2); 2.8194 (0.6); 2.8100 (0.8); 2.8012 (1.2); 2.7914 (1.2); 2.7827 (0.8); 2.7732 (0.6); 2.6708 (0.4); 2.5961 (16.0); 2.5235 (0.8); 2.5101 (20.8); 2.5061 (42.5); 2.5017 (56.7); 2.4973 (41.2); 2.0747 (0.7); 0.7041 (0.7); 0.6913 (2.0); 0.6861 (2.9); 0.6741 (2.6); 0.6682 (2.3); 0.6571 (0.9); 0.5542 (1.0); 0.5438 (3.0); 0.5375 (2.6); 0.5279 (2.4); 0.5158 (0.7); 0.0076 (2.3); -0.0002 (63.7); -0.0084 (2.6) |
| Beispiel 1-T 1 - 12: ¹H-NMR(400.0 MHz, d₆-DMSO): |
| δ= 8.5643 (10.2); 8.3084 (3.2); 8.2986 (3.3); 8.2151 (10.4); 8.0821 (16.0); 7.9803 (7.3); 7.6416 (7.2); 3.9294 (0.8); 3.3239 (34.4); 2.8354 (0.3); 2.8251 (1.0); 2.8159 (1.5); 2.8070 (2.2); 2.7975 (2.2); 2.7888 (1.5); 2.7794 (1.0); 2.7690 (0.4); 2.6719 (0.5); 2.5066 (70.2); 2.5027 (90.6); 2.4990 (68.6); 2.3299 (0.5); 1.0695 (4.9); 0.7210 (1.2); 0.7080 (3.8); 0.7032 (5.2); 0.6910 (4.8); 0.6853 (4.2); 0.6742 (1.7); 0.5698 (1.7); 0.5593 (5.3); 0.5528 (5.1); 0.5438 (4.4); 0.5313 (1.2); 0.0000 (9.6) |
| Beispiel 1-T 1 - 13: ¹H-NMR(400.0 MHz, d₆-DMSO): |
| δ= 9.2167 (6.6); 8.5961 (10.8); 8.2406 (10.8); 8.0864 (16.0); 8.0779 (8.4); 8.0746 (8.1); 7.6590 (7.6); 7.6557 (7.5); 4.0560 (0.8); 4.0382 (2.4); 4.0204 (2.5); 4.0027 (0.8); 3.9282 (0.4); 3.3221 (44.4); 2.6756 (0.6); 2.6712 (0.8); 2.6666 (0.6); 2.5245 (1.9); 2.5110 (48.0); 2.5066 (99.8); 2.5021 (132.6); 2.4976 (94.4); 2.4933 (44.7); 2.3332 (0.5); 2.3288 (0.8); 2.3242 (0.5); 1.9891 (10.7); 1.5862 (2.4); 1.5720 (5.9); 1.5650 (6.3); 1.5521 (2.7); 1.3975 (1.5); 1.2853 (2.9); 1.2719 (5.9); 1.2651 (6.4); 1.2507 (2.4); 1.2353 (0.6); 1.1927 (2.8); 1.1749 (5.7); 1.1571 (2.8); 1.0690 (2.7); 0.0080 (0.6); -0.0001 (18.2); -0.0083 (0.6) |
| Beispiel 1-T 1 - 14: ¹H-NMR(400.0 MHz, d₆-DMSO): |
| δ= 8.6192 (0.6); 8.5622 (0.5); 8.5430 (10.1); 8.2947 (3.2); 8.2839 (3.1); 8.2419 (0.8); 8.1934 (10.2); 8.0812 (16.0); 7.3909 (0.4); 7.3014 (10.6); 5.7567 (1.3); 3.4248 (2.4); 2.8374 (0.3); 2.8272 (0.9); 2.8179 (1.4); 2.8091 (2.0); 2.7996 (2.0); 2.7903 (1.4); 2.7812 (1.0); 2.7709 (0.4); 2.6699 (0.8); 2.5012 (151.5); 2.3281 (0.9); 0.7256 (1.1); 0.7122 (3.7); 0.7077 (4.9); 0.6952 (4.6); 0.6897 (4.0); 0.6786 (1.6); 0.5697 (1.6); 0.5591 (5.1); 0.5521 (4.9); 0.5435 (4.3); 0.5315 (1.2); 0.1455 (0.6); 0.0058 (5.7); -0.0004 (125.5); -0.0019 (107.6); -0.1505 (0.6) |
| Beispiel 1-T 1 - 15: ¹H-NMR(400.0 MHz, d₆-DMSO): |
| δ= 9.1972 (6.6); 8.5625 (10.4); 8.2010 (10.6); 8.0850 (16.0); 7.3696 (11.4); 6.9083 (0.7); 5.7566 (4.4); 3.3216 (25.6); 2.6712 (0.6); 2.5065 (76.8); 2.5023 (100.2); 2.4982 (74.5); 2.3292 (0.7); 2.0749 (2.1); 1.6008 (2.3); 1.5866 (6.0); 1.5798 (6.5); 1.5668 (2.7); 1.2777 (2.8); 1.2643 (6.2); 1.2576 (6.5); 1.2425 (2.9); 1.2233 (1.3); 1.1916 (0.8); 1.1752 (0.8); 0.8810 (0.8); 0.8641 (0.8); 0.0075 (2.4); -0.0001 (56.4) |
| Beispiel 1-T 1 - 16: ¹H-NMR(400.0 MHz, d₆-DMSO): |
| δ= 9.1968 (0.4); 8.7769 (1.0); 8.7466 (0.4); 8.5761 (9.3); 8.5621 (0.6); 8.3702 (2.5); 8.3595 (2.6); 8.2724 (1.1); 8.2292 (9.3); 8.2007 (0.6); 8.0858 (16.0); 7.8646 (0.6); 7.4156 (10.1); 7.3687 (0.6); 5.7567 (12.1); 3.3217 (86.6); 2.8437 (0.4); 2.8330 (1.0); 2.8241 (1.4); 2.8148 (2.0); 2.8052 (2.0); 2.7964 (1.3); 2.7863 (0.9); 2.7770 (0.4); 2.6712 (0.9); 2.5062 (129.2); 2.5020 (170.7); 2.4979 (125.2); 2.3284 (0.9); 2.0744 (2.6); 1.5860 (0.4); 1.5797 (0.4); 1.2624 (0.4); 1.2569 (0.4); 0.7379 (0.5); |
| 0.7270 (1.4); 0.7146 (3.3); 0.7093 (4.5); 0.6971 (4.3); 0.6913 (3.6); 0.6802 (1.5); 0.5629 (1.5); 0.5522 (4.5); 0.5455 (4.3); 0.5363 (3.9); 0.5240 (1.4); 0.5069 (0.5); 0.1463 (0.3); 0.0077 (2.5); -0.0002 (68.7); -0.1495 (0.3) |
| Beispiel 1-T 1 - 17: ¹H-NMR(400.0 MHz, d₆-DMSO): |
| δ= 9.6859 (1.5); 9.2607 (5.2); 8.8019 (2.3); 8.5964 (8.8); 8.2949 (2.4); 8.2366 (8.9); 8.0890 (16.0); 7.4870 (10.3); 3.3210 (33.2); 2.6752 (0.5); 2.6709 (0.7); 2.6665 (0.5); 2.5240 (1.7); 2.5104 (42.9); 2.5062 (89.1); 2.5018 (119.6); 2.4974 (86.3); 2.4932 (41.6); 2.3289 (0.6); 2.3237 (0.5); 1.6520 (0.5); 1.6379 (1.3); 1.6310 (1.3); 1.6175 (0.6); 1.5987 (1.9); 1.5843 (4.8); 1.5774 (5.1); 1.5644 (2.2); 1.2817 (2.2); 1.2682 (4.8); 1.2615 (5.2); 1.2469 (2.4); 1.2329 (1.4); 1.2262 (1.4); 1.2118 (0.5); 0.9264 (0.3); 0.9097 (0.3); 0.0077 (1.6); -0.0004 (42.8); -0.0086 (1.6) |
| Beispiel 1-T 1 - 18: ¹H-NMR(400.0 MHz, d₆-DMSO): |
| δ= 8.8156 (1.0); 8.6100 (9.3); 8.3161 (1.2); 8.2652 (8.4); 8.0870 (16.0); 7.3933 (12.2); 5.7553 (1.7); 3.3194 (128.0); 3.0327 (13.4); 2.9944 (2.9); 2.6749 (1.2); 2.6706 (1.8); 2.6665 (1.3); 2.5240 (4.5); 2.5104 (109.0); 2.5061 (227.3); 2.5017 (304.5); 2.4973 (220.5); 2.4934 (108.7); 2.3327 (1.3); 2.3285 (1.7); 2.3244 (1.3); 2.0739 (0.4); 2.0084 (0.4); 1.9891 (0.4); 1.7318 (0.4); 1.7190 (0.5); 1.7076 (0.4); 1.6376 (2.3); 1.4846 (0.4); 1.4441 (2.3); 1.4305 (4.3); 1.4245 (4.4); 1.4085 (1.4); 1.2354 (3.1); 0.8542 (0.7); 0.1463 (0.8); 0.0079 (5.5); -0.0001 (165.6); -0.0084 (7.0); -0.1495 (0.8) |

| | |
|---|---|
| 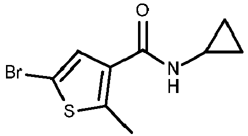 | Intermediat (B): ¹H-NMR(400.0 MHz, d₆-DMSO): |
| | δ= 8.0939 (2.6); 8.0851 (2.6); 7.3625 (16.0); 3.3216 (15.5); 2.7946 (0.5); 2.7845 (1.4); 2.7749 (2.0); 2.7663 (3.1); 2.7563 (3.2); 2.7478 (2.0); 2.7382 (1.5); 2.7280 (0.5); 2.5532 (49.0); 2.5242 (0.7); 2.5106 (16.9); 2.5065 (35.2); 2.5020 (47.1); 2.4976 (34.3); 2.4935 (16.9); 0.6823 (1.8); 0.6699 (5.2); 0.6645 (7.2); 0.6525 (6.9); 0.6465 (5.6); 0.6353 (2.6); 0.6141 (0.4); 0.5962 (0.4); 0.5698 (0.3); 0.5596 (0.4); 0.5303 (2.6); 0.5198 (7.4); 0.5131 (6.6); 0.5099 (6.2); 0.5035 (5.8); 0.4916 (1.8); -0.0002 (8.9); -0.0085 (0.4) |
| 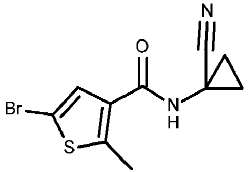 | Inermediat (C): Z-2: ¹H-NMR(400.0 MHz, d₆-DMSO): |
| | δ= 8.9557 (2.0); 7.4046 (5.3); 3.3226 (6.4); 2.5921 (16.0); 2.5069 (12.6); 2.5024 (16.8); 2.4980 (12.3); 1.5434 (1.1); 1.5294 (3.0); 1.5222 (3.0); 1.5092 (1.4); 1.2531 (1.4); 1.2399 (3.0); 1.2328 (3.2); 1.2187 (1.1); -0.0002 (3.5) |

### Biologische Beispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis*, wenn in diesem Test bei einer Aufwandmenge von 5 µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 µg/cm² (= 500g/ha): 1, 11, 12

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus*, wenn in diesem Test bei einer Aufwandmenge von 5 µg/cm² mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 5 µg/cm² (= 500g/ha): 1, 2, 3, 11, 12, 14

Bei diesem Test zeigen z.B. die folgenden Verbindungen der Herstellungsbeispiele eine Wirkung von 80% bei einer Aufwandmenge von 5 µg/cm² (= 500g/ha): 15

### Boophilus microplus - Diptest

| | |
|---|---|
| Testtiere: | Rinderzecken (*Boophilus microplus)* Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: 1, 2, 11, 14

### Boophilus microplus -Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: 1, 2, 3, 4, 6, 7, 10, 11, 13, 14, 15

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: 1, 2, 3, 4, 5, 6, 8, 10, 11, 12, 13, 14, 15

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 1, 2, 3, 4, 10, 11, 12, 13, 14, 15

Bei diesem Test zeigen z.B. die folgenden Verbindungen der Herstellungsbeispiele eine Wirkung von 95% bei einer Aufwandmenge von 100ppm: 6

Bei diesem Test zeigen z.B. die folgenden Verbindungen der Herstellungsbeispiele eine Wirkung von 80% bei einer Aufwandmenge von 100ppm: 5, 8

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: 1, 2, 10, 11, 12, 13, 14, 15

Bei diesem Test zeigen z.B. die folgenden Verbindungen der Herstellungsbeispiele eine Wirkung von 90% bei einer Aufwandmenge von 100ppm: 5

### Myzus persicae - Oraltest

| | | |
|---|---|---|
| Lösungsmittel: | 100 | Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus *(Myzus persicae*), die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 4ppm: 10, 12, 16

Bei diesem Test zeigen z. B. die folgenden Verbindungen der Herstellungsbeispiele Wirkung von 90% bei einer Aufwandmenge von 4ppm: 11

### Myzus persicae - Sprühtest

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis),* die von allen Stadien der Grünen Pfirsichblattlaus (*Myzus persicae*) befallen sind, werden mit einer Wirkstoffzubereitung der gewünschten Konzentration gespritzt.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 100g/ha: 1, 2, 16

Bei diesem Test zeigen z. B. die folgenden Verbindungen der Herstellungsbeispiele Wirkung von 90% bei einer Aufwandmenge von 100g/ha: 11

### Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 100g/ha: 1, 2, 3, 4, 5, 9, 10, 11, 12, 13, 14, 15, 16, 17, 18

Bei diesem Test zeigen z. B. die folgenden Verbindungen der Herstellungsbeispiele Wirkung von 83% bei einer Aufwandmenge von 100g/ha: 7, 8

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 100g/ha: 1, 2, 3, 6, 9, 10, 11, 12, 13, 14, 15, 16, 17, 18

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 100g/ha: 10, 11, 13, 14, 15, 16, 17

Bei diesem Test zeigen z. B. die folgenden Verbindungen der Herstellungsbeispiele eine Wirkung von 100% bei einer Aufwandmenge von 20g/ha: 1, 10, 11, 17

Bei diesem Test zeigen z. B. die folgenden Verbindungen der Herstellungsbeispiele eine Wirkung von 90% bei einer Aufwandmenge von 20g/ha: 2, 13, 15, 16

### Anopheles Test (ANPHGB Oberflächenbehandlung)

### Lösungsmittel: Aceton + 2000 ppm Rapsölmethylester (RME)

Zur Herstellung einer zweckmäßigen Wirkstoffzubereitung löst man den Wirkstoff im Lösungsmittel (2 mg/ml). Die Wirkstoffzubereitung wird auf eine lasierte Kachel pipettiert und nach Abtrocknen werden adulte Mücken der Spezies Anopheles gambiae Stamm RSPH (homozygot kdr) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 80-100% bei einer Aufwandmenge von 100 mg/m²: 1, 2, 3, 8, 6, 9, 10, 14, 15.

### Anopheles Test (ANPHFU Oberflächenbehandlung)

### Lösungsmittel: Aceton + 2000 ppm Rapsölmethylester (RME)

Zur Herstellung einer zweckmäßigen Wirkstoffzubereitung löst man den Wirkstoff im Lösungsmittel (2 mg/ml). Die Wirkstoffzubereitung wird auf eine lasierte Kachel pipettiert und nach Abtrocknen werden adulte Mücken der Spezies Anopheles funestus Stamm FUMOZ-R (Hunt et al., Med Vet Entomol. 2005 Sep; 19(3):271-5) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 80-100% bei einer Aufwandmenge von 100 mg/m²: 1, 2, 9, 10, 14, 15.

### Aedes Test (AEDSAE Oberflächenbehandlung)

### Lösungsmittel: Aceton + 2000 ppm Rapsölmethylester (RME)

Zur Herstellung einer zweckmäßigen Wirkstoffzubereitung löst man den Wirkstoff im Lösungsmittel (2 mg/ml). Die Wirkstoffzubereitung wird auf eine lasierte Kachel pipettiert und nach Abtrocknen werden adulte Mücken der Spezies Aedes aegypti Stamm MONHEIM auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 80-100% bei einer Aufwandmenge von 100 mg/m²: 1, 2, 9, 10, 13, 14, 15.

### Absetzbeispiele

### Phaedon cochleariae - Sprühtest (PHAECO)

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle II

### Spodoptera frugiperda - Sprühtest (SPODFR)

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele überlegene Wirksamkeit gegenüber dem Stand der Technik: siehe Tabelle II

### Myzus persicae - Sprühtest (MYZUPE)

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle II

### Tetranychus urticae - Sprühtest ; OP-resistent (TETRUR)

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele überlegene Wirksamkeit gegenüber dem Stand der Technik: siehe Tabelle II

**Tabelle II**

| Substanz | Struktur | Objekt | Konzentration | % Wirkung dat | |
|---|---|---|---|---|---|
| Beispiel-Nr. 36 WO2012/000896 | 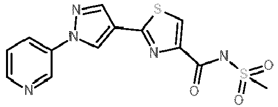 | PHAECO | 500 g ai/ha | 0 | 7 dat |
| | | SPODFR | 500 g ai/ha | 0 | 7 dat |
| | | MYZUPE | 100 g ai/ha | 0 | 6 dat |
| | | TETRUR | 500 g ai/ha | 0 | 6 dat |
| Beispiel-Nr. I-T1-1 erfindungsgemäß | 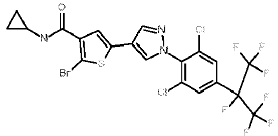 | PHAECO | 100 g ai/ha | 100 | 7 dat |
| | | SPODFR | 100 g ai/ha | 100 | 7 dat |
| | | MYZUPE | 100 g ai/ha | 100 | 5 dat |
| | | TETRUR | 100 g ai/ha | 70 | 6 dat |

Die nachstehenden Ausführungen 1 bis 17 sind ebenfalls Gegenstand der Erfindung.
Ausführung 1: Verbindungen der allgemeine Formel (I) 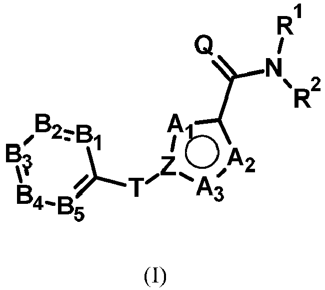 worin
   - R¹: für H, jeweils gegebenenfalls mit einem oder mehreren Substitutenten unabhängig voneinander ausgewählt aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden, einer Oxogruppe oder einer Iminogruppe substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl C₆-,C₁₀-,C₁₄-Aryl(C₁-C₃)-alkyl, 3, 4, 5, 6, 7, 8, 9 oder 10-gliedrigen-Heterocyclyl (z. B. Thietanyl wie Thiethanyl-3-yl, Oxidothiethan wie 1-Oxido-thietan-3-yl, oder Dioxidothiethan wie 1,1-Dioxido-thietan-3-yl) oder 3, 4, 5, 6, 7, 8, 9 oder 10-gliedrigen-Heterocyclyl(C₁-C₃)-alkyl steht,
   bevorzugt für gegebenenfalls mit Halogen oder Cyano substituiertem C₃-C₇-Cycloalkyl, oder
   C₆-,C₁₀-,C₁₄-Aryl(C₁-C₃)-alkyl wobei C₆-,C₁₀-,C₁₄-Aryl gegebenenfalls mit einem oder mehreren Substitutenten unabhängig voneinander ausgewählt aus Amino, Hydroxy, Halogen, Nitro, Cyano, Isocyano, Mercapto und C₁-C₄-Alkyl substituiert sein kann, oder
   C₁-C₆-Alkylcarbonyl;
   mehr bevorzugt für gegebenenfalls mit Cyano substituiertem C₃-Cycloalkyl oder C₆-Aryl(C₁-C₃)-alkyl;
   - R²: für H oder gegebenenfalls mit einem oder mehreren Substitutenten unabhängig voneinander ausgewählt aus Amino, Hydroxy, Halogen, Nitro, Cyano, Mercapto, C₁-C₄-Carboxy, Carbonamid, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden oder einer Oxogruppe substituiertes C₁-C₆-Alkyl steht;
   die Gruppierungen
   - A₁, A₂ und A₃: unabhängig voneinander für N, O, CR³, S oder N-R⁴, bevorzugt für N, CR³, S oder N-R⁴ stehen, wobei A₁, A₂, A₃, Z und das C-Atom des Ringes ein aromatisches System bilden;
   - R³: jeweils unabhängig voneinander für H, Cl, F, I, Br oder gegebenenfalls halogeniertes C₁-C₄-Alkyl, bevorzugt Methyl steht;
   - R⁴: jeweils unabhängig für H oder gegebenenfalls halogeniertes C₁-C₄-Alkyl, bevorzugt Methyl steht;
   - Q: für O oder S, bevorzugt O steht;
   - Z: für C oder N steht; bevorzugt für C steht;
   - B₁ und B₅: unabhängig voneinander für N oder C-R⁵ und
   - B₂, B₃ und B₄: unabhängig voneinander für C-R⁵ stehen, wobei
   - R⁵: unabhängig voneinander für H, Halogen, Cyano, Nitro, SF₅, jeweils gegebenenfalls mit einem oder mehreren Substitutenten ausgewählt aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden, einer Oxogruppe oder einer Iminogruppe substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, steht;
   - T: für einen der nachfolgend aufgeführten 5-gliedrigen Heteroaromaten T1-T4 und T6 steht, wobei die Bindung zu 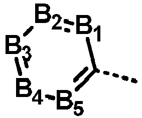 mit einer Raute # gekennzeichnet ist, 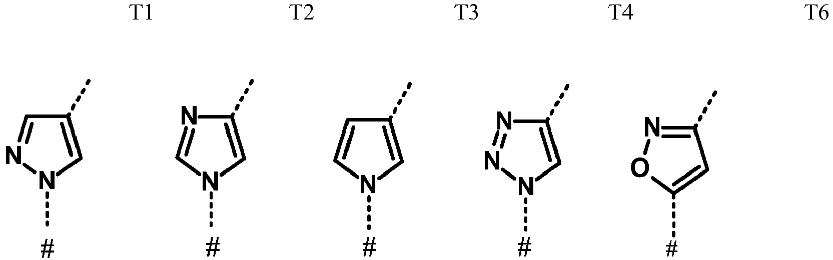 sowie Salze, N-Oxide und tautomere Formen der Verbindungen der Formel (I).
Ausführung 2: Verbindung nach Ausführung 1, wobei R² für H oder Methyl steht.
Ausführung 3: Verbindung nach einer der vorherigen Ausführungen, wobei T für T1, T2 oder T4 steht, besonders bevorzugt für T1 steht.
Ausführung 4: Verbindung nach einer der vorherigen Ausführungen, wobei R¹ für Benzyl, Cyclopropyl oder 1-CN-Cyclopropyl steht.
Ausführung 5: Verbindung nach einer der vorherigen Ausführungen, wobei die Verbindung eine Verbindung gemäß einer der Formeln der Formel (Ia), (Ib), (Ic), (Id) oder (1f) bis (1k) ist. 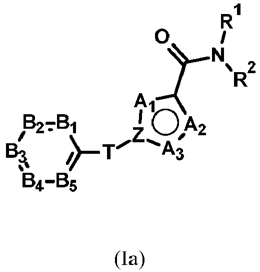 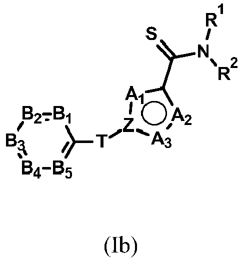 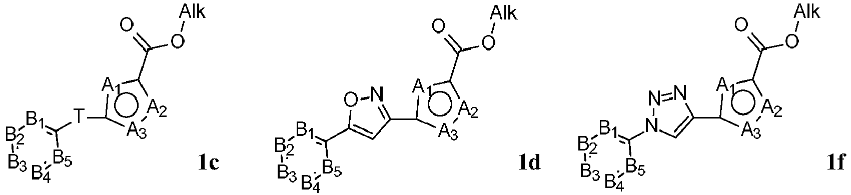 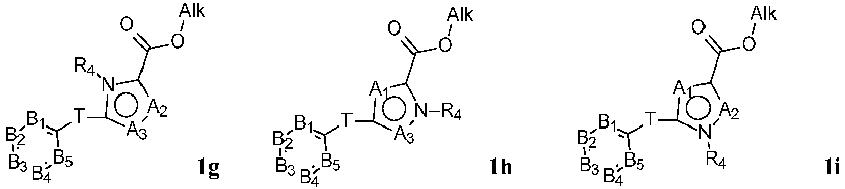 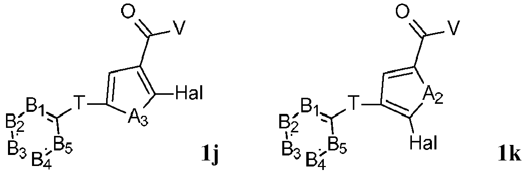
Ausführung 6: Verbindung nach einer der vorherigen Ausführungen, wobei die Verbindung eine Verbindung der Formel (I'a) ist. 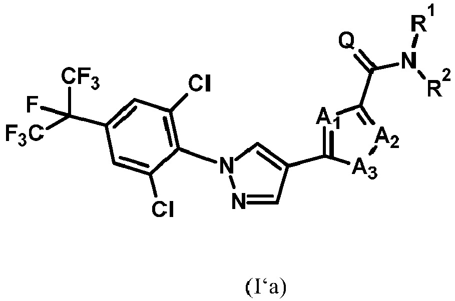
Ausführung 7: Verbindung nach einer der vorherigen Ausführungen, wobei die Verbindung eine Verbindung der Formel (I'b) ist. 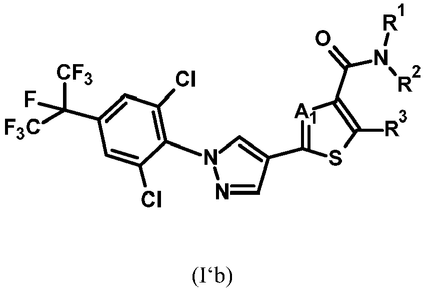
Ausführung 8: Verbindung nach einer der vorherigen Ausführungen, wobei die Verbindung eine Verbindung der Formel (I'c) ist. 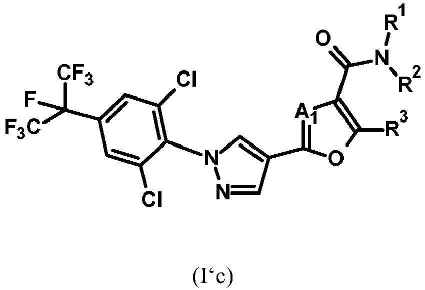
Ausführung 9: Verbindung nach einer der vorherigen Ausführungen, wobei die Verbindung eine Verbindung der Formel (I'd) ist. 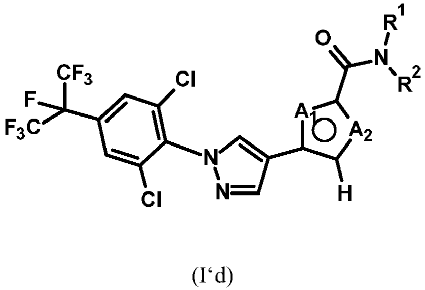
Ausführung 10: Verbindungen nach einer der vorherigen Ausführungen, dadurch gekennzeichnet, dass B₂ und B₄ jeweils für C-H stehen.
Ausführung 11: Verbindungen nach einer der vorherigen Ausführungen, dadurch gekennzeichnet, dass B₃ für CR⁵ und R⁵ für perhalogeniertes C₁-C₄-Alkyl steht.
Ausführung 12: Verbindungen nach Ausführung 11, dadurch gekennzeichnet, dass R⁵ für perfluoriertes C₁-C₄-Alkyl, bevorzugt für perfluoriertes Propyl steht.
Ausführung 13: Insektizides Mittel umfassend mindestens eine Verbindung der Formel (I) nach einer der Ausführungen 1 bis 8 und einem Streckmittel und/oder einer oberflächenaktiven Substanz.
Ausführung 14: Verfahren zum Schutz von transgenem oder konventionellem Saatgut und der daraus entstehenden Pflanze vor dem Befall von Schädlingen, dadurch gekennzeichnet, dass das Saatgut mit mindestens einer Verbindung nach einer der Ausführungen 1 bis 12 behandelt wird.
Ausführung 15: Verwendung von Verbindungen nach einer der Ausführungen 1 bis 12, oder von einem insektiziden Mittel nach Ausführung 13 zum Bekämpfen von Schädlingen.
Ausführung 16: Saatgut, bei dem eine Verbindung gemäß einer der Ausführungen 1 bis 12 als Bestandteil einer Umhüllung oder als weitere Schicht oder weitere Schichten zusätzlich zu einer Umhüllung auf das Saatgut aufgebracht ist.
Ausführung 17: Zwischenverbindungen der Formeln (B) und(C).

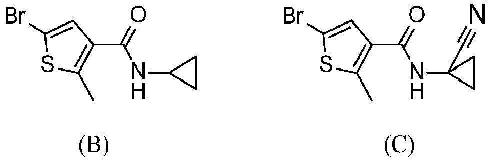

## Patentansprüche

1. Verbindungen der allgemeinen Formel (I) 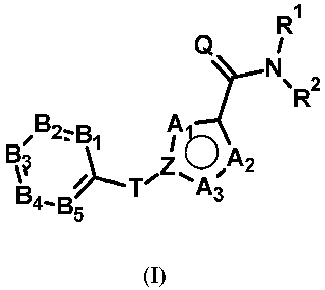 worin
R¹ für H,jeweils gegebenenfalls mit einem oder mehreren Substitutenten unabhängig voneinander ausgewählt aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, N-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulflnylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *N,N*-Di*-*C₁*-*C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden, einer Oxogruppe oder einer Iminogruppe substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, C₆-,C₁₀-,C₁₄-Aryl(C₁-C₃)-alkyl, 3, 4, 5, 6, 7, 8, 9 oder 10-gliedrigen-Heterocyclyl z. B. Thietanyl wie Thiethanyl-3-yl, Oxidothiethan wie 1-Oxido-thietan-3-yl, oder Dioxidothiethan wie 1,1-Dioxido-thietan-3-yl) oder 3, 4, 5, 6, 7, 8, 9 oder 10-gliedrigen-Heterocyclyl(C₁-C₃)-alkyl steht,
bevorzugt für gegebenenfalls mit Halogen oder Cyano substituiertem C₃-C₇-Cycloalkyl, oder
C₆-,C₁₀-,C₁₄-Aryl(C₁-C₃)-alkyl wobei C₆-,C₁₀-,C₁₄-Aryl gegebenenfalls mit einem oder mehreren Substitutenten unabhängig voneinander ausgewählt aus Amino, Hydroxy, Halogen, Nitro, Cyano, Isocyano, Mercapto und C₁-C₄-Alkyl substituiert sein kann, oder
C₁-C₆-Alkylcarbonyl;
mehr bevorzugt für gegebenenfalls mit Cyano substituiertem C₃-Cycloalkyl oder C₆-Aryl(C₁-C₃)-alkyl;
R² für H oder gegebenenfalls mit einem oder mehreren Substitutenten unabhängig voneinander ausgewählt aus Amino, Hydroxy, Halogen, Nitro, Cyano, Mercapto, C₁-C₄-Carboxy, Carbonamid, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, *N*-C₁-C₄-Alkyl-aminocarbonyl, *NN*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden oder einer Oxogruppe substituiertes C₁-C₆-Alkyl steht;
die Gruppierungen
A₁, A₂ und A₃ unabhängig voneinander für N, O, CR³, S oder N-R⁴, bevorzugt für N, CR³, S oder N-R⁴ stehen, wobei A₁, A₂, A₃, Z und das C-Atom des Ringes ein aromatisches System bilden;
R³ jeweils unabhängig voneinander für H, Cl, F, I, Br oder gegebenenfalls halogeniertes C₁-C₄-Alkyl, bevorzugt Methyl steht;
R⁴ jeweils unabhängig für H oder gegebenenfalls halogeniertes C₁-C₄-Alkyl, bevorzugt Methyl steht;
Q für O oder S, bevorzugt O steht;
Z für C oder N steht; bevorzugt für C steht;
B₁ und B₅ unabhängig voneinander für N oder C-R⁵ und
B₂, B₃ und B₄ unabhängig voneinander für C-R⁵ stehen, wobei
R⁵ unabhängig voneinander für H, Halogen, Cyano, Nitro, SF₅, jeweils gegebenenfalls mit einem oder mehreren Substitutenten ausgewählt aus Amino, Hydroxy, Halogen, Nitro, Cyano, Isocyano, Mercapto, Isothiocyanato, C₁-C₄-Carboxy, Carbonamid, SF₅, Aminosulfonyl, C₁-C₄-Alkyl, C₃-C₄-Cycloalkyl, C₂-C₄-Alkenyl, C₅-C₆-Cycloalkenyl, C₂-C₄-Alkinyl, *N*-Mono-C₁-C₄-alkyl-amino, *N,N*-Di-C₁-C₄-alkylamino, *N*-C₁-C₄-Alkanoylamino, C₁-C₄-Alkoxy, C₂-C₄-Alkenyloxy, C₂-C₄-Alkinyloxy, C₃-C₄-Cycloalkoxy, C₅-C₆-Cycloalkenyloxy, C₁-C₄-Alkoxycarbonyl, C₂-C₄-Alkenyloxycarbonyl, C₂-C₄-Alkinyloxycarbonyl, C₆-,C₁₀-,C₁₄-Aryloxycarbonyl, C₁-C₄-Alkanoyl, C₂-C₄-Alkenylcarbonyl, C₂-C₄-Alkinylcarbonyl, C₆-,C₁₀-,C₁₄-Arylcarbonyl, C₁-C₄-Alkylsulfanyl, C₃-C₄-Cycloalkylsulfanyl, C₁-C₄-Alkylthio, C₂-C₄-Alkenylthio, C₅-C₆-Cycloalkenylthio, C₂-C₄-Alkinylthio, C₁-C₄-Alkylsulfenyl und C₁-C₄-Alkylsulfinyl, wobei beide Enantiomere der C₁-C₄-Alkylsulfinylgruppe umfasst sind, C₁-C₄-Alkylsulfonyl, *N*-Mono-C₁-C₄-alkyl-aminosulfonyl, *N,N*-Di-C₁-C₄-alkyl-aminosulfonyl, C₁-C₄-Alkylphosphinyl, C₁-C₄-Alkylphosphonyl, wobei für C₁-C₄-Alkylphosphinyl bzw. C₁-C₄-Alkylphosphonyl beide Enantiomere umfasst sind, N-C₁-C₄-Alkyl-aminocarbonyl, *N,N*-Di-C₁-C₄-alkyl-amino-carbonyl, N-C₁-C₄-Alkanoyl-amino-carbonyl, N-C₁-C₄-Alkanoyl-N-C₁-C₄-alkyl-aminocarbonyl, C₆-,C₁₀-,C₁₄-Aryl, C₆-,C₁₀-,C₁₄-Aryloxy, Benzyl, Benzyloxy, Benzylthio, C₆-,C₁₀-,C₁₄-Arylthio, C₆-,C₁₀-,C₁₄-Arylamino, Benzylamino, Heterocyclyl und Trialkylsilyl, mit einer Doppelbindung verbundenen Substituenten wie C₁-C₄-Alkyliden, einer Oxogruppe oder einer Iminogruppe substituiertes C₁-C₆-Alkyl, C₃-C₆-Cycloalkyl, C₁-C₆-Alkoxy, N-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-D₁-C₁-C₆-alkylamino, steht;
T für einen der nachfolgend aufgeführten 5-gliedrigen Heteroaromaten T2-T4 und T6 steht, wobei die Bindung zu 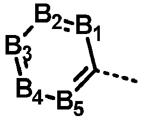 mit einer Raute # gekennzeichnet ist, 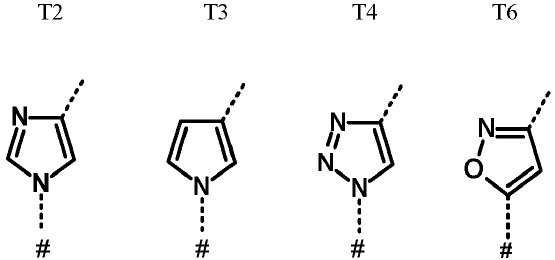 sowie Salze, N-Oxide und tautomere Formen der Verbindungen der Formel (1).

2. Verbindung nach Anspruch 1, wobei R² für H oder Methyl steht.

3. Verbindung nach einem der vorherigen Ansprüche, wobei T für T2, T4 oder T6 steht, weiter bevorzugt für T4 oder T6 steht, besonders bevorzugt für T4 steht.

4. Verbindung nach einem der vorherigen Ansprüche, wobei R¹ für Benzyl, Cyclopropyl oder 1-CN-Cyclopropyl steht.

5. Verbindung nach einem der vorherigen Ansprüche, wobei die Verbindung eine Verbindung gemäß einer der Formeln der Formel (la), (1b), (1c), (1d) oder (1f) bis (1k) ist.

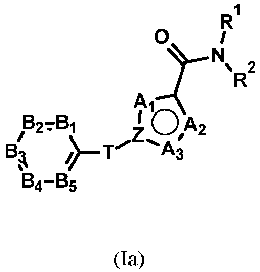

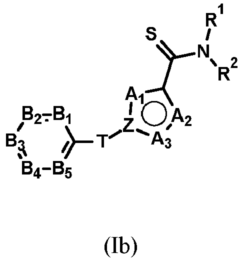

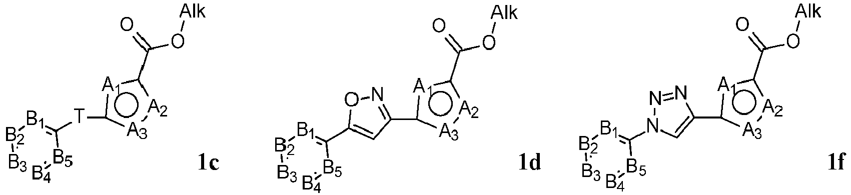

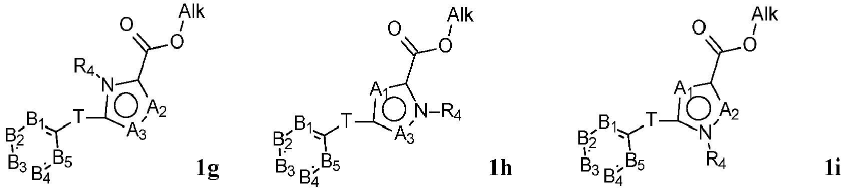

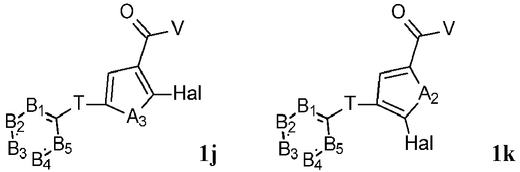

6. Verbindungen nach einem der vorherigen Ansprüche, **dadurch gekennzeichnet, dass** B₂ und B₄ jeweils für C-H stehen.

7. Verbindungen nach einem der vorherigen Ansprüche, **dadurch gekennzeichnet, dass** B₃ für CR⁵ und R⁵ für perhalogeniertes C₁-C₄-Alkyl steht.

8. Verbindungen nach Anspruch 7, **dadurch gekennzeichnet, dass** R⁵ für perfluoriertes C₁-C₄-Alkyl, bevorzugt für perfluoriertes Propyl steht.

9. Insektizides Mittel umfassend mindestens eine Verbindung der Formel (1) nach einem der Ansprüche 1 bis 5 und einem Streckmittel und/oder einer oberflächenaktiven Substanz.

10. Verfahren zum Schutz von transgenem oder konventionellem Saatgut und der daraus entstehenden Pflanze vor dem Befall von Schädlingen, **dadurch gekennzeichnet, dass** das Saatgut mit mindestens einer Verbindung nach einem der Ansprüche 1 bis 8 behandelt wird.

11. Verwendung von Verbindungen nach einem der Ansprüche 1 bis 8, oder von einem insektiziden Mittel nach Anspruch 9 zum Bekämpfen von Schädlingen.

12. Saatgut, bei dem eine Verbindung gemäß einem der Ansprüche 1 bis 8 als Bestandteil einer Umhüllung oder als weitere Schicht oder weitere Schichten zusätzlich zu einer Umhüllung auf das Saatgut aufgebracht ist.

13. Verfahren zur Herstellung von Verbindungen der Formel (la) aus Verbindungen der Formel 1, umfassend die Hydrolyse von Verbindungen der Formel 1 zu Intermediaten der Formel 2 und die Umsetzung der Intermediate 2 mit Aminen der allgemeinen Struktur 3 zu Verbindungen der Formel (la), wobei die Reste A₁, A₂, A₃, Z, T, B₁, B₂, B₃, B₄, B₅, R₁, und R₂ wie in Anspruch 1 definiert sind und Alk für ein C₁-C₄-Alkyl steht.

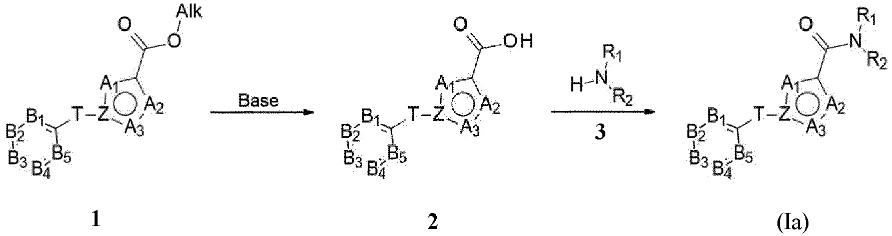

14. Verfahren zur Herstellung von Verbindungen der Formel (Ib) aus Verbindungen der Formel (la), umfassend die Reaktion von Verbindungen der Formel (la) mit einem Schwefelungsreagenz.
